# EUROPEAN PATENT APPLICATION

(11) **EP 2 962 692 A1**
(43) Date of publication of application: **06.01.2016**
(21) Application number: 14757271.3
(22) Date of filing: 03.03.2014
(51) Int. Cl.: A61K 31/519, A61P 27/02, A61P 37/08, C07D 487/04

(54) **AGENT FOR PREVENTING AND/OR TREATING OCULAR INFLAMMATORY DISEASE**

(30) Priority: 01.03.2013 JP 2013040245
(71) Applicant: Kyowa Hakko Kirin Co., Ltd., Tokyo 100-8185 (JP)
(72) Inventor: AONO, Yukiko, Tokyo 100-8185 (JP); KOBAYASHI, Katsuya, Tokyo 100-8185 (JP)
(74) Representative: Vossius & Partner Patentanwälte Rechtsanwälte mbB
(86) International application number: PCT/JP2014/055263
(87) International publication number: WO 2014/133182

(57) **Abstract**

The present invention provides a prophylactic and/or therapeutic agent and the like for ocular inflammatory diseases, comprising a pyrazolopyrimidine compound represented by the following formula (I)
{wherein R¹ represents -NR^{1a}R^{1b} (wherein R^{1a} and R^{1b} are the same or different and each represents a hydrogen atom and the like), and the like,
R² represents the following formula (R²-1) [wherein k and m represent each an integer of 0 - 2, n represents an integer of 0 - 2, L represents a single bond and the like, R⁵ represents a halogen and the like, R⁶ represents aryl and the like, X represents -CR⁸ (wherein R⁸ represents a hydrogen atom and the like), and the like, R⁷ represents a hydrogen atom and the like, and the like,
R³ represents -SO₂R¹³ (wherein R¹³ represents lower alkoxy and the like), and the like,
R⁴ represents a hydrogen atom and the like} or a pharmaceutically acceptable salt thereof, as an active ingredient.

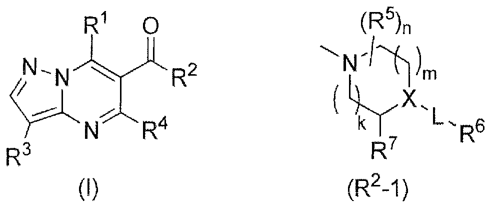

## Description

### Technical Field

The present invention relates to a prophylactic and/or therapeutic agent for ocular inflammatory diseases, comprising a pyrazolopyrimidine compound or a pharmaceutically acceptable salt thereof as an active ingredient, and the like.

### Background Art

Ocular inflammatory diseases are in the forms of many ocular disorders accompanying various pains depending on the site of inflammation. Examples of the ocular inflammatory disease include uveitis, conjunctivitis, cyclitis, scleritis, episcleritis, optic neuritis, retrobulbar optic neuritis, keratitis, blepharitis, corneal ulcer, conjunctiva ulcer and the like (visual ophthalmologic definitive diagnosis, handbook of diagnosis and treatment, the first and second volumes, Medical Review Co., Ltd., 1990). Ocular inflammatory diseases result from various ocular disorders, eye surgery or physical trauma to the eye. Also, the symptom of ocular inflammatory disease includes itching, redness, edema, ulcer and the like. Patients with ocular inflammatory disease account for the majority of patients with ophthalmic disease, and a medicament having an anti-ocular inflammatory action plays an important role in clinical practice. Currently, to suppress inflammation in ocular inflammatory diseases, steroids, non-steroidal antiinflammatory agents or immunosuppressants are used. Also, for allergic conjunctival diseases, antiallergic agents are used in addition to them [Allergology International, 2011, vol. 60, pages 191 - 203].

In the meantime, a pyrazolopyrimidine compound useful as an agent for the prevention and/or treatment of skin diseases is known (patent documents 1 and 2).

Also, a chemokine receptor activity modulator comprising a pyrazolopyrimidine compound as an active ingredient is known (patent document 3)

### Prior Arts Documents

### patent documents

patent document 1: WO 2011/108689
patent document 2: WO 2009/041663
patent document 3: WO 2013/031931

### SUMMARY OF THE INVENTION

### Problems to be Solved by the Invention

An object of the present invention is to provide a prophylactic and/or therapeutic agent for ocular inflammatory diseases (for example, eyelid dermatitis, keratoconjunctivitis, scleritis, conjunctivitis {for example, allergic conjunctival diseases [for example, atopic keratoconjunctivitis, spring catarrh, giant papillary conjunctivitis, allergic conjunctivitis (for example, seasonal allergic conjunctivitis such as pollinosis and the like, perennial allergic conjunctivitis and the like) and the like] and the like} and the like) and the like.

### Means of Solving the Problems

The present invention relates to the following (1) - (54).
(1) A prophylactic and/or therapeutic agent for ocular inflammatory diseases, comprising a pyrazolopyrimidine compound represented by the formula (I) 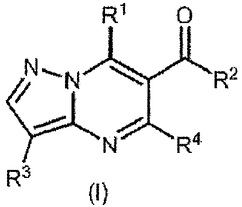 (wherein R¹ represents -NR^{1a}R^{1b} (wherein R^{1a} and R^{1b} are the same or different and each represents a hydrogen atom, lower alkyl optionally having substituent(s), cycloalkyl optionally having substituent(s), lower alkoxy optionally having substituent(s), lower alkanoyl optionally having substituent(s), aryl optionally having substituent(s), aralkyl optionally having substituent(s), an aromatic heterocyclic group optionally having substituent(s) or an aliphatic heterocyclic group optionally having substituent(s), or R^{1a} and R^{1b} form, together with the adjacent nitrogen atom, a nitrogen-containing heterocyclic group optionally having substituent(s)), -OR^{1c} (wherein R^{1c} represents a hydrogen atom, lower alkyl optionally having substituent(s), cycloalkyl optionally having substituent(s), aryl optionally having substituent(s), aralkyl optionally having substituent(s), an aromatic heterocyclic group optionally having substituent(s) or an aliphatic heterocyclic group optionally having substituent(s)) or -SR^{1d} (wherein R^{1d} is as defined for the aforementioned R^{1c}), R² represents 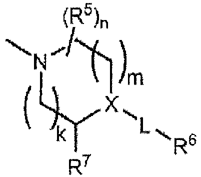
   [wherein k and m each represents an integer of 0 - 2 (wherein the total of k and m is not more than 3),
   n represents an integer of 0 - 4, and when n is 2, 3 or 4, respective R⁵ may be the same or different,
   L represents a single bond, alkylene, C(=O) or SO₂,
   R⁵ represents halogen, hydroxy, lower alkyl optionally having substituent(s) or lower alkoxy optionally having substituent(s),
   R⁶ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
   X represents a nitrogen atom or -CR⁸ (wherein R⁸ represents a hydrogen atom, halogen, hydroxy, cyano, lower alkyl optionally having substituent(s) or lower alkoxy optionally having substituent(s), or forms a bond together with R⁷), and R⁷ forms a bond together with R⁸, or represents a hydrogen atom, halogen, hydroxy, lower alkyl optionally having substituent(s) or lower alkoxy optionally having substituent(s)], 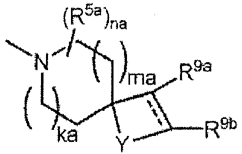
      {wherein ka, ma and na are as defined for the aforementioned k, m and n, respectively,
      R^{5a} is as defined for the aforementioned R⁵,
      --- represents a single bond or a double bond,
      R^{9a} and R^{9b} are the same or different and each represents a hydrogen atom or lower alkyl optionally having substituent(s), or R^{9a} and R^{9b} form, together with the respectively adjacent carbon atoms, an aliphatic ring optionally having substituent(s) or an aromatic ring optionally having substituent(s),
      Y represents -CHR^{10a}-CHR^{10b}- (wherein R^{10a} and R^{10b} are the same or different and each represents a hydrogen atom, hydroxy, lower alkyl optionally having substituent(s) or lower alkoxy optionally having substituent(s), or R^{10a} and R^{10b} form, together with the respectively adjacent carbon atoms, an aliphatic ring optionally having substituent (s)), -CR^{10c}=CR^{10d}- (wherein R^{10c} and R^{10d} are the same or different and each represents a hydrogen atom or lower alkyl optionally having substituent(s), or R^{10c} and R^{10d} form, together with the respectively adjacent carbon atoms, an aliphatic ring having at least one double bond and optionally having substituent(s) or an aromatic ring optionally having substituent(s)), -Z^{a} -CR^{11a}R^{11b}- [wherein R^{11a} and R^{11b} are the same or different and each represents a hydrogen atom or lower alkyl optionally having substituent(s), or R^{11a} and R^{11b} form carbonyl together with the adjacent carbon atom, and Z^{a} represents C(=O), 0, S, SO, SO₂ or NR¹² (wherein R¹² represents a hydrogen atom, lower alkyl optionally having substituent(s), lower alkanoyl optionally having substituent(s), lower alkoxycarbonyl optionally having substituent(s) or aralkyl optionally having substituent(s))], or -CR^{11c}R^{11d}-Z^{b}- (wherein R^{11c}, R^{11d} and Z^{b} are as defined for the aforementioned R^{11a}, R^{11b} and Z^{a}, respectively)}, or 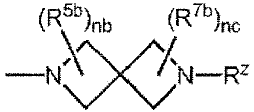 (wherein R^{z} represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s), R^{5b} and R^{7b} are the same or different and each represents halogen, hydroxy, lower alkyl optionally having substituent(s) or lower alkoxy optionally having substituent(s), nb represents an integer of 0 - 2, nc represents an integer of 0 - 2, when nb is 2, respective R^{5b}s are the same or different, when nc is 2, respective R^{7b}s are the same or different, R³ represents -S(O)₂R^{13a} [wherein R^{13a} represents hydroxy, lower alkoxy optionally having substituent (s), -NR^{13b}R^{13c} (wherein R^{13b} and R^{13c} are the same or different and each represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), or R^{13b} and R^{13c} form, together with the adjacent nitrogen atom, a nitrogen-containing heterocyclic group optionally having substituent(s)), -NR^{13d}C(=O)R^{13e} (wherein R^{13d} represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), and R^{13e} represents a hydrogen atom, lower alkyl optionally having substituent(s), cycloalkyl optionally having substituent(s), aralkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s), N,N-di-arylamino optionally having substituent(s), aryl optionally having substituent(s), an aromatic heterocyclic group optionally having substituent(s) or an aliphatic heterocyclic group optionally having substituent (s)), -NR^{13f}C (=S) R^{13g} (wherein R^{13f} and R^{13g} are each as defined for the aforementioned R^{13d} and R^{13e}, respectively), or-NR^{13h}S(O)₂R¹⁴ (wherein R^{13h} is as defined for the aforementioned R^{13d}, and R¹⁴ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s))], carboxy, lower alkoxycarbonyl optionally having substituent(s), lower alkyl optionally having substituent(s), lower alkanoyl optionally having substituent(s), aralkyl optionally having substituent(s), an aromatic heterocyclic group optionally having substituent(s), -C(=O)NR^{23a}R^{23b} [wherein R^{23a} and R^{23b} are the same or different and each represents a hydrogen atom, lower alkyl optionally having substituent(s), lower alkanoyl optionally having substituent(s), aralkyl optionally having substituent (s) or -S(O)₂R²⁴ (wherein R²⁴ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s)), or R^{23a} and R^{23b} form, together with the adjacent nitrogen atom, a nitrogen-containing heterocyclic group optionally having substituent(s)], or -NR^{23c}R^{23d} (wherein R^{23c} and R^{23d} are each as defined for the aforementioned R^{23a} and R^{23b}, respectively), R⁴ represents a hydrogen atom, halogen, lower alkyl optionally having substituent(s), aralkyl optionally having substituent(s), -NR^{15a}R^{15b} (wherein R^{15a} and R^{15b} are the same or different and each represents a hydrogen atom, lower alkyl optionally having substituent(s), cycloalkyl optionally having substituent(s), lower alkoxy optionally having substituent(s), lower alkanoyl optionally having substituent(s), aryl optionally having substituent(s), aralkyl optionally having substituent(s), an aromatic heterocyclic group optionally having substituent(s) or an aliphatic heterocyclic group optionally having substituent(s), or R^{15a} and R^{15b} form, together with the adjacent nitrogen atom, a nitrogen-containing heterocyclic group optionally having substituent(s)), -OR^{15c} (wherein R^{15c} represents a hydrogen atom, lower alkyl optionally having substituent(s), cycloalkyl optionally having substituent(s), aryl optionally having substituent(s), aralkyl optionally having substituent(s), an aromatic heterocyclic group optionally having substituent(s) or an aliphatic heterocyclic group optionally having substituent(s)), or -SR^{15d} (wherein R^{15d} is as defined for the aforementioned R^{15c})) or a pharmaceutically acceptable salt thereof, as an active ingredient.
(2) The prophylactic and/or therapeutic agent according to the aforementioned (1), wherein R³ is -S(O)₂R^{13a} [wherein R^{13a} represents hydroxy, lower alkoxy optionally having substituent(s), -NR^{13b}R^{13c} (wherein R^{13b} and R^{13c} are the same or different and each represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), or R^{13b} and R^{13c} form, together with the adjacent nitrogen atom, a nitrogen-containing heterocyclic group optionally having substituent(s)), -NR^{13d}C(=O)R^{13e} (wherein R^{13d} represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), and R^{13e} represents a hydrogen atom, lower alkyl optionally having substituent(s), cycloalkyl optionally having substituent(s), aralkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s), N,N-di-arylamino optionally having substituent(s), aryl optionally having substituent(s), an aromatic heterocyclic group optionally having substituent(s) or an aliphatic heterocyclic group optionally having substituent(s)), -NR^{13f}C (=S) R^{13g} (wherein R^{13f} and R^{13g} are as defined for the aforementioned R^{13d} and R^{13e}, respectively), or-NR^{13h}S(O)₂R¹⁴ (wherein R^{13h} is as defined for the aforementioned R^{13d}, and R¹⁴ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s))].
(3) A prophylactic and/or therapeutic agent for ocular inflammatory diseases, comprising a pyrazolopyrimidine compound represented by the formula (IA) 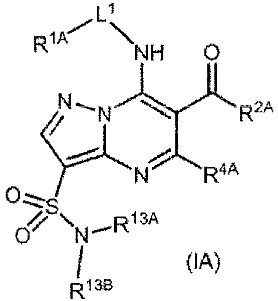 (wherein
   L¹ represents a single bond or methylene,
   R^{1a} represents lower alkyl optionally having substituent(s), aralkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
   R^{2A} represents 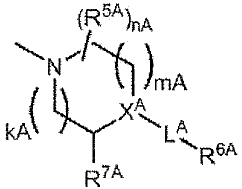
      [wherein nA represents an integer of 0 - 2, and when nA is 2, respective R^{5A}s may be the same or different,
      kA, mA and L^{A} are as defined for the aforementioned k, m and L, respectively,
      R^{5A} represents halogen or lower alkyl,
      R^{6A} represents cycloalkyl optionally having substituent(s), aryl optionally having substituent(s), an aromatic heterocyclic group optionally having substituent(s) or an aliphatic heterocyclic group optionally having substituent(s), X^{A} is a nitrogen atom or -CR^{8A} (wherein R^{8A} represents a hydrogen atom, halogen or lower alkyl, or forms a bond together with R^{7A}), and
      R^{7A} forms a bond together with R^{8A}, or represents a hydrogen atom, halogen or lower alkyl], 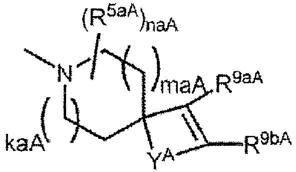
         {wherein naA and R^{5aA} are as defined for the aforementioned nA and R^{5A}, respectively,
         maA and kaA are as defined for the aforementioned ma and ka, respectively,
         R^{9aA} and R^{9bA} form, together with the respectively adjacent carbon atoms, an aromatic ring optionally having substituent(s), Y^{A} represents -CHR^{10aA}-CHR^{10bA}- (wherein R^{10aA} and R^{10bA} are the same or different and each represents a hydrogen atom, hydroxy, lower alkyl optionally having substituent(s) or lower alkoxy optionally having substituent (s)), -CR^{10cA}=CR^{10dA}- (wherein R^{10cA} and R^{10dA} are the same or different and each represents a hydrogen atom or lower alkyl optionally having substituent(s)), -Z^{aA}-CR^{11aA}R^{11bA}- [wherein R^{11aA} and R^{11bA} are the same or different and each represents a hydrogen atom or lower alkyl optionally having substituent(s), or R^{11aA} and R^{111bA} form carbonyl together with the adjacent carbon atom, and Z^{aA} represents C(=O), O, S, SO, SO₂ or NR^{12A} (wherein R^{12A} represents a hydrogen atom, lower alkyl optionally having substituent(s), lower alkanoyl optionally having substituent(s), lower alkoxycarbonyl optionally having substituent(s) or aralkyl optionally having substituent(s))], or -CR^{11cA}R^{11dA}-Z^{bA}- (wherein R^{11cA}, R^{11dA} and Z^{bA} are as defined for the aforementioned R^{11aA}, R^{11bA} and Z^{aA}, respectively)}, or 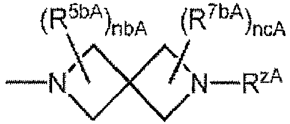 (wherein R^{zA} represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s), R^{5bA} and R^{7bA} are the same or different and each represents halogen, hydroxy, lower alkyl optionally having substituent(s) or lower alkoxy optionally having substituent(s), nbA represents an integer of 0 - 2, ncA represents an integer of 0 - 2, when nbA is 2, respective R^{5bA}s are the same or different and when ncA is 2, respective R^{7bA}s are the same or different),
            R^{13A} represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s),
            R^{13B} represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s), an aliphatic heterocyclic group optionally having substituent(s) or COR^{13e1} (wherein R^{13e1} is as defined for the aforementioned R^{13e}), or R^{13B} and R^{13A} form, together with the adjacent nitrogen atom, a nitrogen-containing heterocyclic group optionally having substituent(s),
            R^{4A} represents a hydrogen atom or lower alkyl optionally having substituent(s)) or a pharmaceutically acceptable salt thereof, as an active ingredient.
(4) The prophylactic and/or therapeutic agent according to the aforementioned (3), wherein L¹ is a single bond.
(5) The prophylactic and/or therapeutic agent according to the aforementioned (3) or (4), wherein R^{1A} is aryl optionally having substituent(s).
(6) The prophylactic and/or therapeutic agent according to the aforementioned (3) or (4), wherein R^{1A} is phenyl optionally having substituent(s).
(7) The prophylactic and/or therapeutic agent according to any of the aforementioned (3) - (6), wherein R^{4A} is a hydrogen atom.
(8) The prophylactic and/or therapeutic agent according to any of the aforementioned (3) - (7), wherein R^{13A} is a hydrogen atom, and R^{13B} is lower alkyl optionally having substituent(s).
(9) The prophylactic and/or therapeutic agent according to any of the aforementioned (3) - (7), wherein R^{13A} is a hydrogen atom, and R^{13B} is COR^{13e1} (wherein R^{13e1} is as defined above) .
(10) The prophylactic and/or therapeutic agent according to the aforementioned (9), wherein R^{13e1} is lower alkyl optionally having substituent(s), cycloalkyl optionally having substituent(s), N-mono-lower alkylamino optionally having substituent(s) or N-mono-arylamino optionally having substituent(s).
(11) The prophylactic and/or therapeutic agent according to any of the aforementioned (3) - (10), wherein R^{2A} is 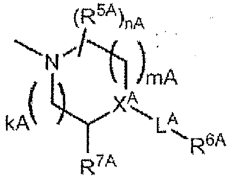 (wherein nA, mA, kA, R^{5A}, R^{6A}, R^{7A}, L^{A} and X^{A} are each as defined above, respectively).
(12) The prophylactic and/or therapeutic agent according to the aforementioned (11), wherein R^{6A} is phenyl optionally having substituent(s).
(13) The prophylactic and/or therapeutic agent according to the aforementioned (12), wherein nA is 0, kA and mA are each 1, R^{7A} is a hydrogen atom, L^{A} is a single bond, and X^{A} is CH.
(14) The prophylactic and/or therapeutic agent according to any of the aforementioned (3) - (10), wherein R^{2A} is 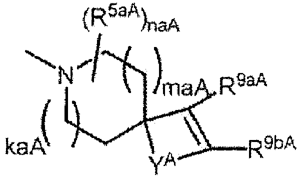 (wherein naA, maA, kaA, R^{5aA}, R^{9aA}, R^{9bA} and Y^{A} are each as defined above, respectively).
(15) The prophylactic and/or therapeutic agent according to the aforementioned (14), wherein R^{9aA} and R^{9bA} form, together with the respectively adjacent carbon atoms, a benzene ring optionally having substituent(s), Y^{A} is -CHR^{10aA}-CHR^{10bA}- (wherein R^{10aA} and R^{10bA} are each as defined above, respectively),-CR^{10cA}=CR^{10dA}- (wherein R^{10cA} and R^{10dA} are each as defined above, respectively), -O-CR^{11aA}R^{11bA}- (wherein R^{11aA} and R^{11bA} are each as defined above, respectively), or -CR^{11cA}R^{11dA}-O- (wherein R^{11cA} and R^{11dA} are each as defined above, respectively).
(16) The prophylactic and/or therapeutic agent according to any of the aforementioned (3) - (10), wherein R^{2A} is 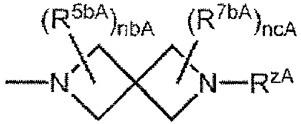 (wherein R^{zA}, R^{5bA}, R^{7bA}, nbA and ncA are each as defined above, respectively).
(17) The prophylactic and/or therapeutic agent according to the aforementioned (16), wherein R^{zA} is phenyl optionally having substituent(s).
(18) The prophylactic and/or therapeutic agent according to the aforementioned (1), wherein R¹ is -NHR^{1b}, said R^{1b} is phenyl optionally having substituent(s) selected from the group consisting of halogen and lower alkyl,
   R² is 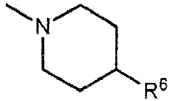 or 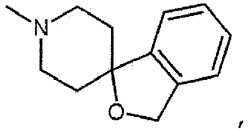 said R⁶ is phenyl optionally having halogen,
   R³ is -S(O)₂R^{13a} or -C (=O) NH-S(O)₂-R²⁴, said R^{13a} is -NHR^{13c} or-NHC(=O)R^{13e}, said R^{13c} is lower alkoxy, said R^{13e} is lower alkyl or N-mono-lower alkylamino, and said R²⁴ is lower alkyl.
(19) The prophylactic and/or therapeutic agent according to the aforementioned (18), wherein R^{1b} is phenyl optionally having substituent(s) selected from the group consisting of a fluorine atom, a chlorine atom and methyl.
(20) The prophylactic and/or therapeutic agent according to the aforementioned (18), wherein R^{1b} is phenyl having two substituents selected from the group consisting of a fluorine atom, a chlorine atom and methyl.
(21) The prophylactic and/or therapeutic agent according to any of the aforementioned (18) - (21), wherein R² is 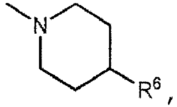 and said R⁶ is phenyl optionally having halogen.
(22) The prophylactic and/or therapeutic agent according to the aforementioned (21), wherein R⁶ is phenyl optionally having a fluorine atom.
(23) The prophylactic and/or therapeutic agent according to any of the aforementioned (18) - (22), wherein R³ is -S(O)₂R^{13a}, said R^{13a} is -NHR^{13c} or -NHC(=O)R^{13e}, said R^{13c} is lower alkoxy, and said R^{13e} is lower alkyl or N-mono-lower alkylamino.
(24) The prophylactic and/or therapeutic agent according to the aforementioned (23), wherein R^{13c} is C₁₋₄ alkoxy, and R^{13e} is C₁₋₄ alkyl or N-mono-C₁₋₄ alkylamino.
(25) The prophylactic and/or therapeutic agent according to any of the aforementioned (18) - (22), wherein R³ is -C(=O)NH-S(O)₂-R²⁴, and said R²⁴ is lower alkyl.
(26) The prophylactic and/or therapeutic agent according to the aforementioned (25), wherein R²⁴ is C₁₋₄ alkyl.
(27) The prophylactic and/or therapeutic agent according to the aforementioned (18), wherein the pyrazolopyrimidine compound is a compound selected from the group consisting of
   7-(2-chloro-5-methylphenylamino)-N-(ethylcarbamoyl)-6-[4-(4-fluorophenyl)piperidine-1-carbonyl]pyrazolo[1,5-a]pyrimidine-3-sulfonamide,
   N-{7-(2,5-dichlorophenylamino)-6-[4-(4-fluorophenyl)piperidine-1-carbonyl]pyrazolo[1,5-a]pyrimidin-3-ylsulfonyl}propionamide,
   7-(2,5-dichlorophenylamino)-6-[4-(4-fluorophenyl)piperidine-1-carbonyl]-N-methoxypyrazolo[1,5-a]pyrimidine-5-sulfonamide,
   N-{7-(5-chloro-2-fluorophenylamino)-6-[4-(4-fluorophenyl)piperidine-1-carbonyl]pyrazolo[1,5-a]pyrimidin-3-ylsulfonyl}acetamide,
   7-(5-chloro-2-fluorophenylamino)-N-(ethylcarbamoyl)-6-[4-(4-fluorophenyl)piperidine-1-carbonyl]pyrazolo[1,5-a]pyrimidine-3-sulfonamide,
   N-{7-(2,5-difluorophenylamino)-6-[4-(4-fluorophenyl)piperidine-1-carbonyl]pyrazolo[1,5-a]pyrimidin-3-ylsulfonyl}acetamide,
   7-(2-chloro-5-methylphenylamino)-N-(ethylcarbamoyl)-6-(3H-spiro[isobenzofuran-1,4'-piperidin]-1'-yl carbonyl)pyrazolo[1,5-a]pyrimidine-3-sulfonamide,
   N-[7-(4-fluoro-2-methylphenylamino)-6-(4-phenylpiperidine-1-carbonyl)pyrazolo[1,5-alpyrimidine-3-carbonyl]ethanesulfonamide,
   N-{7-(2-fluoro-5-methylphenylamino)-6-[4-(4-fluorophenyl)piperidine-1-carbonyl]pyrazolo[1,5-a]pyrimidine-3-carbonyl}ethanesulfonamide, and
   N-{7-(4-fluoro-2-methylphenylamino)-6-[4-(4-fluorophenyl)piperidine-1-carbonyl]pyrazolo[1,5-a]pyrimidine-3-carbonyl}ethanesulfonamide.
(28) The prophylactic and/or therapeutic agent according to any of the aforementioned (1) - (27), wherein the ocular inflammatory disease is a disease selected from conjunctivitis, eyelid dermatitis, keratoconjunctivitis and scleritis.
(29) The prophylactic and/or therapeutic agent of any according to the aforementioned (1) - (27), wherein the ocular inflammatory disease is conjunctivitis.
(30) The prophylactic and/or therapeutic agent according to the aforementioned (29), wherein the conjunctivitis is an allergic conjunctival disease.
(31) The prophylactic and/or therapeutic agent according to the aforementioned (30), wherein the allergic conjunctival disease is a disease selected from allergic conjunctivitis, atopic keratoconjunctivitis, spring catarrh and giant papillary conjunctivitis.
(32) The prophylactic and/or therapeutic agent according to the aforementioned (31), wherein the allergic conjunctivitis is a disease selected from seasonal allergic conjunctivitis and perennial allergic conjunctivitis.
(33) The prophylactic and/or therapeutic agent according to any of the aforementioned (1) - (27), which is used for itching, redness, edema and ulcer occurring as symptoms of ocular inflammatory diseases.
(34) A method for the prophylaxis and/or treatment of an ocular inflammatory disease, comprising a step of administering an effective amount of the pyrazolopyrimidine compound according to any of the aforementioned (1) - (27) or a pharmaceutically acceptable salt thereof.
(35) The method according to the aforementioned (34), wherein the ocular inflammatory diseases is a disease selected from conjunctivitis, eyelid dermatitis, keratoconjunctivitis and scleritis.
(36) The method according to the aforementioned (35), wherein the ocular inflammatory disease is conjunctivitis.
(37) The method according to the aforementioned (36), wherein the conjunctivitis is an allergic conjunctival disease.
(38) The method according to the aforementioned (37), wherein the allergic conjunctival disease is a disease selected from allergic conjunctivitis, atopic keratoconjunctivitis, spring catarrh and giant papillary conjunctivitis.
(39) The method according to the aforementioned (38), wherein the allergic conjunctivitis is a disease selected from seasonal allergic conjunctivitis and perennial allergic conjunctivitis.
(40) A method for the prophylaxis and/or treatment of itching, redness, edema and ulcer occurring as symptoms of ocular inflammatory diseases, comprising a step of administering an effective amount of the pyrazolopyrimidine compound according to any of the aforementioned (1) - (27) or a pharmaceutically acceptable salt thereof.
(41) The pyrazolopyrimidine compound according to any of the aforementioned (1) - (27) or a pharmaceutically acceptable salt thereof for use in the prophylaxis and/or treatment of ocular inflammatory diseases.
(42) The pyrazolopyrimidine compound according to the aforementioned (41) or a pharmaceutically acceptable salt thereof, wherein the ocular inflammatory diseases is a disease selected from conjunctivitis, eyelid dermatitis, keratoconjunctivitis and scleritis.
(43) The pyrazolopyrimidine compound according to the aforementioned (41) or a pharmaceutically acceptable salt thereof, wherein the ocular inflammatory diseases is conjunctivitis.
(44) The pyrazolopyrimidine compound according to the aforementioned (43) or a pharmaceutically acceptable salt thereof, wherein the conjunctivitis is an allergic conjunctival disease.
(45) The pyrazolopyrimidine compound according to the aforementioned (44) or a pharmaceutically acceptable salt thereof, wherein the allergic conjunctival disease is a disease selected from allergic conjunctivitis, atopic keratoconjunctivitis, spring catarrh and giant papillary conjunctivitis.
(46) The pyrazolopyrimidine compound according to the aforementioned (45) or a pharmaceutically acceptable salt thereof, wherein the allergic conjunctivitis is a disease selected from seasonal allergic conjunctivitis and perennial allergic conjunctivitis.
(47) The pyrazolopyrimidine compound according to any of the aforementioned (1) - (27) or a pharmaceutically acceptable salt thereof for use in the prophylaxis and/or treatment of itching, redness, edema and ulcer occurring as symptoms of ocular inflammatory diseases.
(48) Use of the pyrazolopyrimidine compound according to any of the aforementioned (1) - (27) or a pharmaceutically acceptable salt thereof for the manufacture of a prophylactic and/or therapeutic agent for ocular inflammatory diseases.
(49) The use according to the aforementioned (48), wherein the ocular inflammatory disease is a disease selected from conjunctivitis, eyelid dermatitis, keratoconjunctivitis and scleritis.
(50) The use according to the aforementioned (48), wherein the ocular inflammatory disease is conjunctivitis.
(51) The use according to the aforementioned (50), wherein the conjunctivitis is an allergic conjunctival disease.
(52) The use according to the aforementioned (51), wherein the allergic conjunctival disease is a disease selected from allergic conjunctivitis, atopic keratoconjunctivitis, spring catarrh and giant papillary conjunctivitis.
(53) The use according to the aforementioned (52), wherein the allergic conjunctivitis is a disease selected from seasonal allergic conjunctivitis and perennial allergic conjunctivitis.
(54) Use of the pyrazolopyrimidine compound according to any of the aforementioned (1) - (27) or a pharmaceutically acceptable salt thereof for the manufacture of a prophylactic and/or therapeutic agent for itching, redness, edema and ulcer occurring as symptoms of ocular inflammatory diseases.

### Effect of the Invention

According to the present invention, a prophylactic and/or therapeutic agent for ocular inflammatory diseases and the like comprising a pyrazolopyrimidine compound represented by the formula (I) or (IA) or a pharmaceutically acceptable salt thereof as an active ingredient are provided.

The prophylactic and/or therapeutic agent for ocular inflammatory diseases to be provided by the present invention is useful for the treatment and/or prophylaxis of ocular inflammatory diseases (for example, eyelid dermatitis, keratoconjunctivitis, scleritis, conjunctivitis {for example, allergic conjunctival diseases [for example, atopic keratoconjunctivitis, spring catarrh, giant papillary conjunctivitis, allergic conjunctivitis (for example, seasonal allergic conjunctivitis such as pollinosis and the like, perennial allergic conjunctivitis and the like) and the like] and the like} and the like).

### Mode for Carrying Out the Invention

In the present specification, compounds represented by the formula (I) and the formula (IA) are referred to as compound (I) and compound (IA), respectively. This also applies to compounds having other formula numbers.

In the definition of each group in the formulas (I) and (IA),
examples of the lower alkyl, and the lower alkyl moiety of the lower alkoxy, the lower alkoxycarbonyl, the lower alkanoyl, the N,N-di-lower alkylaminomethyleneamino, the N-mono-lower alkylamino and the N,N-di-lower alkylamino include linear or branched alkyl having 1 - 10 carbon atoms, and more specific examples thereof include methyl, ethyl, propyl, isopropyl, butyl, isobutyl, sec-butyl, tert-butyl, pentyl, isopentyl, neopentyl, hexyl, heptyl, octyl, nonyl, decyl and the like. The two lower alkyl moieties of the N,N-di-lower alkylamino and the N,N-di-lower alkylaminomethyleneamino may be the same or different.

Alkylene has the same meaning as the group formed by removing one hydrogen atom from the aforementioned lower alkyl.

Examples of the cycloalkyl, and the cycloalkyl moiety of the N-cycloalkylamino include cycloalkyl having 3 - 8 carbon atoms, and more specific examples thereof include cyclopropyl, cyclobutyl, cyclopentyl, cyclohexyl, cycloheptyl, cyclooctyl and the like.

Examples of the aryl, and the aryl moiety of the N-mono-arylamino and the N,N-di-arylamino include aryl having 6 - 14 carbon atoms, and more specific examples thereof include phenyl, naphthyl, azulenyl, anthryl, pentalenyl, indenyl, biphenylenyl and the like. The two aryl moieties of the N,N-di-arylamino may be the same or different.

The alkylene moiety of aralkyl has the same meaning as the group formed by removing one hydrogen atom from the aforementioned lower alkyl, and the aryl moiety is as defined for the aforementioned aryl.

Examples of the aliphatic heterocyclic group include a 3-to 7-membered monocyclic aliphatic heterocyclic group containing at least one atom selected from a nitrogen atom, an oxygen atom and a sulfur atom, a bicyclic or tricyclic fused aliphatic heterocyclic group containing at least one atom selected from a nitrogen atom, an oxygen atom and a sulfur atom, wherein 3- to 8-membered rings are fused, and the like, and more specific examples thereof include aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolinyl, oxiranyl, tetrahydrofuranyl, tetrahydro-2H-pyranyl, 5,6-dihydro-2H-pyranyl, oxazolidinyl, morpholino, morpholinyl, thioxazolidinyl, thiomorpholinyl, 2H-oxazolyl, 2H-thioxazolyl, dihydroindolyl, dihydroisoindolyl, dihydrobenzofuranyl, benzimidazolidinyl, dihydrobenzooxazolyl, dihydrobenzothioxazolyl, benzodioxolinyl, tetrahydroquinolyl, tetrahydroisoquinolyl, dihydro-2H-chromanyl, dihydro-1H-chromanyl, dihydro-2H-thiochromanyl, dihydro-1H-thiochromanyl, tetrahydroquinoxalinyl, tetrahydroquinazolinyl, dihydrobenzodioxanyl, oxetanyl and the like.

Examples of the aromatic heterocyclic group include a 5-membered or 6-membered monocyclic aromatic heterocyclic group containing at least one atom selected from a nitrogen atom, an oxygen atom and a sulfur atom, a bicyclic or tricyclic fused aromatic heterocyclic group containing at least one atom selected from a nitrogen atom, an oxygen atom and a sulfur atom, wherein 3- to 8-membered rings are fused, and the like, and more specific examples thereof include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyridazinyl, pyrimidinyl, pyrazinyl, triazinyl, benzofuranyl, benzothiophenyl, benzoxazolyl, benzothiazolyl, isoindolyl, indolyl, indazolyl, benzimidazolyl, benzotriazolyl, oxazolopyrimidinyl, thiazolopyrimidinyl, pyrrolopyridinyl, pyrrolopyrimidinyl, imidazopyridinyl, purinyl, quinolinyl, isoquinolinyl, cinnolinyl, phthalazinyl, quinazolinyl, quinoxalinyl, naphthyridinyl, azepinyl, diazepinyl, pyranyl, oxepinyl, thiopyranyl, thiepinyl, furazanyl, oxadiazolyl, oxazinyl, oxadiazinyl, oxazepinyl, oxadiazepinyl, thiazinyl, thiadiazinyl, thiazepinyl, thiadiazepinyl, indolizinyl, isobenzofuranyl, isobenzothiophenyl, dithianaphthalenyl, quinolizinyl, pteridinyl, benzoxazolidinyl, chromenyl, benzooxepinyl, benzooxadiazepinyl, benzothiepinyl, benzothiazepinyl, benzothiadiazepinyl, benzothiepinyl, benzothiazepinyl, benzoazepinyl, benzodiazepinyl, benzofurazanyl, benzothiadiazolinyl, carbazolyl, β-carbolinyl, acrydinyl, phenazinyl, dibenzofuranyl, xanthenyl, dibenzothiophenyl, phenothiazinyl, phenoxazinyl, phenoxathinyl, thianthrenyl, phenanthridinyl, phenanthrolinyl, perimidinyl and the like.

Examples of the nitrogen-containing heterocyclic group formed together with the adjacent nitrogen atom thereto include a 5-membered or 6-membered monocyclic heterocyclic group containing at least one nitrogen atom (said monocyclic heterocyclic group may contain other nitrogen atom, oxygen atom or sulfur atom), a bicyclic or tricyclic fused heterocyclic group containing at least one nitrogen atom (said fused heterocyclic group may contain other nitrogen atom, oxygen atom or sulfur atom), wherein 3- to 8-membered rings are fused, and the like, and more specific examples thereof include aziridinyl, azetidinyl, pyrrolidinyl, piperidino, azepanyl, pyrrolyl, imidazolidinyl, imidazolyl, pyrazolidinyl, pyrazolinyl, pyrazolyl, piperazinyl, homopiperazinyl, oxazolidinyl, 2H-oxazolyl, thioxazolidinyl, 2H-thioxazolyl, morpholino, thiomorpholinyl, dihydroindolyl, dihydroisoindolyl, indolyl, isoindolyl, tetrahydroquinolyl, tetrahydroisoquinolyl, dihydrobenzooxazolyl, dihydrobenzothioxazolyl, benzimidazolidinyl, benzimidazolyl, dihydroindazolyl, indazolyl, benzotriazolyl, pyrrolopyridinyl, pyrrolopyrimidinyl, imidazopyridinyl, purinyl and the like.

Examples of the aliphatic ring include aliphatic rings respectively corresponding to the aforementioned cycloalkyl and aliphatic heterocyclic group.

Examples of the aliphatic ring having at least one double bond include those having one or more double bonds from among the aforementioned aliphatic rings. Examples thereof include 1,2,5,6-tetrahydropyridine, tetrahydro-2H-pyran, 2H-oxazoline, 2H-thioxazoline, dihydroindoline, dihydroisoindoline, dihydrobenzofuran, dihydrobenzooxazoline, dihydrobenzothioxazoline, dihydro-2H-chromane, dihydro-1H-chromane, dihydro-2H-thiochromane, dihydro-1H-thiochromane, dihydrobenzodioxane and the like.

Examples of the aromatic ring include those respectively corresponding to the aforementioned aryl and aromatic heterocyclic group.

Halogen means each atom of fluorine, chlorine, bromine or iodine.

The substituents (substituent group-1) in the lower alkyl optionally having substituent(s), the lower alkoxy optionally having substituent(s), the N-mono-lower alkylamino optionally having substituent(s), the N,N-di-lower alkylamino optionally having substituent(s), the lower alkoxycarbonyl optionally having substituent(s) and the lower alkanoyl optionally having substituent(s) are the same or different and examples thereof include 1 to 3 substituents selected from the group consisting of halogen; sulfanyl; nitro; cyano; C₃₋₈ cycloalkyl optionally having 1 to 3 substituents selected from the following substituent group C; an aliphatic heterocyclic group optionally having 1 to 3 substituents selected from the following substituent group C; an aromatic heterocyclic group optionally having 1 to 3 substituents selected from the following substituent group B; C₁₋₁₀ alkylsulfanyl optionally having 1 to 3 substituents selected from the following substituent group A; C₆₋₁₄ arylsulfanyl optionally having 1 to 3 substituents selected from the following substituent group B; C₁₋₁₀ alkylsulfonyl optionally having 1 to 3 substituents selected from the following substituent group A; C₆₋₁₄ arylsulfonyl optionally having 1 to 3 substituents selected from the following substituent group B; OR^{16a} (wherein R^{16a} is a hydrogen atom, C₁₋₁₀ alkyl optionally having 1 to 3 substituents selected from the following substituent group A, C₃₋₈ cycloalkyl optionally having 1 to 3 substituents selected from the following substituent group C, C₆₋₁₄ aryl optionally having 1 to 3 substituents selected from the following substituent group B, C₇₋₁₆ aralkyl optionally having 1 to 3 substituents selected from the following substituent group B, an aromatic heterocyclic group optionally having 1 to 3 substituents selected from the following substituent group B, C₂₋₁₁ alkanoyl optionally having 1 to 3 substituents selected from the following substituent group A, C₇₋₁₅ aroyl optionally having 1 to 3 substituents selected from the following substituent group B, C₁₋₁₀ alkylsulfonyl optionally having 1 to 3 substituents selected from the following substituent group A or C₆₋₁₄ arylsulfonyl optionally having 1 to 3 substituents selected from the following substituent group B); C(=O)R^{17a} (wherein R^{17a} is amino, hydroxy, C₁₋₁₀ alkyl optionally having 1 to 3 substituents selected from the following substituent group A, C₃₋₈ cycloalkyl optionally having 1 to 3 substituents selected from the following substituent group C, C₆₋₁₄ aryl optionally having 1 to 3 substituents selected from the following substituent group B, an aliphatic heterocyclic group optionally having 1 to 3 substituents selected from the following substituent group C, an aromatic heterocyclic group optionally having 1 to 3 substituents selected from the following substituent group B, C₁₋₁₀ alkoxy optionally having 1 to 3 substituents selected from the following substituent group A, C₆₋₁₄ aryloxy optionally having 1 to 3 substituents selected from the following substituent group B, C₁₋₁₀ alkylamino optionally having 1 to 3 substituents selected from the following substituent group A, di-C₁₋₁₀ alkylamino optionally having 1 to 3 substituents selected from the following substituent group A or C₆₋₁₄ arylamino optionally having 1 to 3 substituents selected from the following substituent group B); and -NR^{18a}R^{18b} (wherein R^{18a} and R^{18b} are the same or different and each is a hydrogen atom, formyl, C₁₋₁₀ alkyl optionally having 1 to 3 substituents selected from the following substituent group A, C₃₋₈ cycloalkyl optionally having 1 to 3 substituents selected from the following substituent group C, C₆₋₁₄ aryl optionally having 1 to 3 substituents selected from the following substituent group B, an aliphatic heterocyclic group optionally having 1 to 3 substituents selected from the following substituent group C, an aromatic heterocyclic group optionally having 1 to 3 substituents selected from the following substituent group B, C₂₋₁₁ alkanoyl optionally having 1 to 3 substituents selected from the following substituent group A, C₇₋₁₅ aroyl optionally having 1 to 3 substituents selected from the following substituent group B, C₁₋₁₀ alkoxycarbonyl optionally having 1 to 3 substituents selected from the following substituent group A, C₁₋₁₀ alkylsulfonyl optionally having 1 to 3 substituents selected from the following substituent group A or C₆₋₁₄ arylsulfonyl optionally having 1 to 3 substituents selected from the following substituent group B). The substituent in the lower alkoxy optionally having substituent(s), the lower alkoxycarbonyl optionally having substituent(s) and the lower alkanoyl optionally having substituent(s) may be C₆₋₁₄ aryl optionally having 1 to 3 substituents selected from the following substituent group B in addition to the aforementioned substituent group-1.

The substituents in the aryl optionally having substituent(s), the N-mono-arylamino optionally having substituent(s), the N,N-di-arylamino optionally having substituent(s), the phenyl optionally having substituent(s), the aralkyl optionally having substituent(s), the aromatic heterocyclic group optionally having substituent(s), the aromatic ring optionally having substituent(s) and the benzene ring optionally having substituent(s) are the same or different and examples thereof include 1 to 3 substituents selected from the group consisting of C₁₋₁₀ alkyl optionally having 1 to 3 substituents selected from the following substituent group A, C₆₋₁₄ aryl optionally having 1 to 3 substituents selected from the following substituent group B and the substituents recited as the aforementioned substituent group-1. The substituent in the aryl optionally having substituent(s) also includes, in addition to the above, a ring wherein the aryl moiety is fused with a C₄₋₈ cycloalkyl ring optionally having 1 to 3 substituents selected from the following substituent group C or an aliphatic heterocycle optionally having 1 to 3 substituents selected from the following substituent group C. The substituent in the phenyl optionally having substituent(s) also includes, in addition to the above, a ring wherein the phenyl moiety is fused with a C₄₋₈ cycloalkyl ring optionally having 1 to 3 substituents selected from the following substituent group C or an aliphatic heterocycle optionally having 1 to 3 substituents selected from the following substituent group C. The substituent in the aryl moiety of aralkyl optionally having substituent(s) also includes, in addition to the above, a ring wherein the aryl moiety of aralkyl is fused with a C₄₋₈ cycloalkyl ring optionally having 1 to 3 substituents selected from the following substituent group C or an aliphatic heterocycle optionally having 1 to 3 substituents selected from the following substituent group C.

The substituents in the cycloalkyl optionally having substituent(s), the N-cycloalkylamino optionally having substituent(s), the aliphatic heterocyclic group optionally having substituent(s), the aliphatic ring having at least one double bond and optionally having substituent(s), the nitrogen-containing heterocyclic group optionally having substituent(s), which is formed together with the adjacent nitrogen atom, and the aliphatic ring optionally having substituent(s) are the same or different and examples thereof include 1 to 3 substituents selected from the group consisting of oxo, C₁₋₁₀ alkyl optionally having 1 to 3 substituents selected from the following substituent group A, C₆₋₁₄ aryl optionally having 1 to 3 substituents selected from the following substituent group B and the substituents recited as the aforementioned substituent group-1. The substituent in the cycloalkyl optionally having substituent(s) also includes, in addition to the above, a ring wherein the cycloalkyl moiety is fused with a benzene ring optionally having 1 to 3 substituents selected from the following substituent group B.

Substituent group A means a group consisting of halogen; hydroxy; sulfanyl; nitro; cyano; carboxy; carbamoyl; C₃₋₈ cycloalkyl; C₆₋₁₄ aryl optionally having 1 to 3 substituents selected from the group consisting of halogen, hydroxy, amino, nitro, carboxy, C₁₋₁₀ alkoxycarbonyl, C₁₋₁₀ alkoxy and trifluoromethyl (substituent group a); aliphatic heterocyclic group; aromatic heterocyclic group; C₁₋₁₀ alkoxy optionally having 1 to 3 substituents selected from the group consisting of halogen, hydroxy, amino, carboxy, C₁₋₁₀ alkoxy, C₁₋₁₀ alkylamino, di-C₁₋₁₀ alkylamino and C₁₋₁₀ alkoxycarbonyl (substituent group b); C₃₋₈ cycloalkoxy; C₆₋₁₄ aryloxy optionally having 1 to 3 substituents selected from the aforementioned substituent group a; C₇₋₁₆ aralkyloxy optionally having 1 to 3 substituents selected from the aforementioned substituent group a; C₂₋₁₁ alkanoyloxy; C₇₋₁₅ aroyloxy; C₁₋₁₀ alkylsulfonyloxy; trifluoromethanesulfonyloxy; C₆₋₁₄ arylsulfonyloxy; p-toluenesulfonyloxy; C₁₋₁₀ alkylsulfanyl; C₆₋₁₄ arylsulfanyl;-NR^{19a}R^{19b} (wherein R^{19a} and R^{19b} are the same or different and each is a hydrogen atom, formyl, C₁₋₁₀ alkyl optionally having 1 to 3 substituents selected from the aforementioned substituent group b, C₃₋₈ cycloalkyl, C₆₋₁₄ aryl optionally having 1 to 3 substituents selected from the aforementioned substituent group a, aromatic heterocyclic group, C₇₋₁₆ aralkyl optionally having 1 to 3 substituents selected from the aforementioned substituent group a, C₂₋₁₁ alkanoyl, C₇₋₁₅ aroyl, C₁₋₁₀ alkoxycarbonyl, C₇₋₁₆ aralkyloxycarbonyl, C₁₋₁₀ alkylsulfonyl, trifluoromethanesulfonyl, C₆₋₁₄ arylsulfonyl or p-toluenesulfonyl) ; C₂₋₁₁ alkanoyl; C₃₋₈ cycloalkylcarbonyl; C₇₋₁₅ aroyl; aliphatic heterocyclylcarbonyl; aromatic heterocyclylcarbonyl; C₁₋₁₀ alkoxycarbonyl; C₆₋₁₄ aryloxycarbonyl; C₇₋₁₆ aralkyloxycarbonyl; C₁₋₁₀ alkylcarbamoyl; di-C₁₋₁₀ alkylcarbamoyl and C₆₋₁₄ arylcarbamoyl.

Substituent group B means a group consisting of C₁₋₁₀ alkyl, trifluoromethyl and the substituents recited as the aforementioned substituent group A.

Substituent group C means a group consisting of oxo, C₁₋₁₀ alkyl, trifluoromethyl and the substituents recited as the aforementioned substituent group A.

The C₁₋₁₀ alkyl, and the C₁₋₁₀ alkyl moiety of the C₁₋₁₀ alkoxy, the C₂₋₁₁ alkanoyloxy, the C₁₋₁₀ alkylsulfonyl, the C₂₋₁₁ alkanoyl, the C₁₋₁₀ alkoxycarbonyl, the C₁₋₁₀ alkylcarbamoyl, the di-C₁₋₁₀ alkylcarbamoyl, the C₁₋₁₀ alkylsulfonyl, the C₁₋₁₀ alkylsulfonyloxy, the C₁₋₁₀ alkylamino and the di-C₁₋₁₀ alkylamino shown here is exemplified by, for example, the groups recited as the aforementioned lower alkyl. Two C₁₋₁₀ alkyl moieties of di-C₁₋₁₀ alkylcarbamoyl and di-C₁₋₁₀ alkylamino may be the same or different.

The C₃₋₈ cycloalkyl, and the C₃₋₈ cycloalkyl moiety of the C₃₋₈ cycloalkoxy and the C₃₋₈ cycloalkylcarbonyl is exemplified by, for example, the groups recited as the aforementioned cycloalkyl.

Examples of the aryl fused with a C₄₋₈ cycloalkyl ring and the moiety formed by removing the alkylene moiety from the aralkyl of which the aryl moiety is fused with a C₄₋₈ cycloalkyl ring include a cycloalkyl-fused aryl having 8 to 16 carbon atoms, and more specific examples include indanyl, 1,2,3,4-tetrahydronaphthalenyl and the like.

Examples of the phenyl fused with a C₄₋₈ cycloalkyl ring include a cycloalkyl-fused phenyl having 8 to 12 carbon atoms, and more specific examples include indanyl, 1,2,3,4-tetrahydronaphthalenyl and the like.

Examples of the cycloalkyl fused with a benzene ring include a benzene ring-fused cycloalkyl having 8 to 12 carbon atoms, and more specific examples include indanyl, 1,2,3,4-tetrahydronaphthalenyl and the like.

Examples of the C₆₋₁₄ aryl and the aryl moiety of the C₆₋₁₄ aryloxy, the C₆₋₁₄ arylamino, the C₆₋₁₄ arylsulfanyl, the C₇₋₁₅ aroyl, the C₇₋₁₅ aroyloxy, the C₆₋₁₄ aryloxycarbonyl, the C₆₋₁₄ arylsulfonyl, the C₆₋₁₄ arylsulfonyloxy and the C₆₋₁₄ arylcarbamoyl include the groups exemplified as the aforementioned aryl.

Examples of the aryl moiety of the C₇₋₁₆ aralkyloxy, the C₇₋₁₆ aralkyl and the C₇₋₁₆ aralkyloxycarbonyl include the groups exemplified as the aforementioned aryl, and examples of the alkylene moiety include C₁₋₁₀ alkylene and the like, more specifically, the group formed by removing one hydrogen atom from the group exemplified as the aforementioned lower alkyl, and the like.

Examples of the aliphatic heterocyclic group and the aliphatic heterocyclic group moiety of the aliphatic heterocyclylcarbonyl include the groups exemplified as the aforementioned aliphatic heterocyclic group.

Examples of the aromatic heterocyclic group and the aromatic heterocyclic group moiety of the aromatic heterocyclylcarbanyl include the groups exemplified as the aforementioned aromatic heterocyclic group.

Examples of the aryl fused with an aliphatic heterocycle and the moiety formed by removing the alkylene moiety from the aralkyl of which the aryl moiety is fused with an aliphatic heterocycle include aryl fused with a 4- to 7-membered monocyclic aliphatic heterocyclic group containing at least one atom selected from a nitrogen atom, an oxygen atom and a sulfur atom, and more specific examples include dihydrobenzofuranyl, dihydroisobenzofuranyl, indolinyl, isoindolinyl, chromanyl, isochromanyl and the like.

Examples of the phenyl fused with an aliphatic heterocycle include phenyl fused with a 4- to 7-membered monocyclic aliphatic heterocyclic group containing at least one atom selected from a nitrogen atom, an oxygen atom and a sulfur atom, and more specific examples include dihydrobenzofuranyl, dihydroisobenzofuranyl, indolinyl, isoindolinyl, chromanyl, isochromanyl and the like.

Examples of halogen include the atoms exemplified as the aforementioned halogen.

In each group of compound (I),
R¹ is preferably NR^{1a}R^{1b} (wherein R^{1a} and R^{1b} are each as defined above), more preferably one of R^{1a} and R^{1b} is a hydrogen atom, and the other is lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), and more preferably, one of R^{1a} and R^{1b} is a hydrogen atom and the other is aralkyl optionally having substituent(s) or aryl optionally having substituent(s) (wherein the substituent(s) in the aralkyl optionally having substituent(s) or the aryl optionally having substituent(s) is preferably lower alkyl or halogen and the number thereof is preferably 2 or 3). More preferred is when one of R^{1a} and R^{1b} is a hydrogen atom and the other is an aromatic heterocyclic group optionally having substituent(s).

When R² is

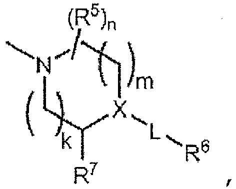

R⁵ is preferably lower alkyl optionally having substituent(s), more preferably lower alkyl.

R⁶ is preferably aryl optionally having substituent(s) or an aromatic heterocyclic group optionally having substituent(s), more preferably aryl optionally having substituent(s), further preferably phenyl optionally having substituent(s).

R⁷ is preferably a hydrogen atom or lower alkyl.

X is preferably a nitrogen atom or CR^{8a} (wherein R^{8a} represents a hydrogen atom, halogen or lower alkyl), more preferably CR^{8a} (wherein R^{8a} is as defined above).

L is preferably a single bond.

k and m are each preferably 1.
n is preferably 0 or 1, more preferably 0.

When R² is

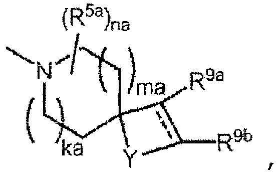

R^{5a} is preferably lower alkyl optionally having substituent(s), more preferably lower alkyl.

R^{9a} and R^{9b} preferably form, together with the respectively adjacent carbon atoms, an aromatic ring optionally having substituent(s), more preferably form, together with the respectively adjacent carbon atoms, a benzene ring optionally having substituent(s).

Y is preferably -CHR^{10a}-CHR^{10b}- (wherein R^{10a} and R^{10b} are each as defined above), -CR^{10e}=CR^{10d}- (wherein R^{10c} and R^{10d} are each as defined above), -O-CR^{1a}R^{11b}- (wherein R^{11a} and R^{11b} are each as defined above), or -CR^{11c}R^{11d}-O- (wherein R^{11c} and R^{11d} are each as defined above), more preferably -CHR^{10aa}-CHR^{10ba}-(wherein R^{10aa} and R^{10ba} are the same or different and each represents a hydrogen atom or lower alkyl), -CR^{10ca}=CR^{10da}-(wherein R^{10ca} and R^{10da} are the same or different and each represents a hydrogen atom or lower alkyl), -O-CR^{11a}R^{11ba}-(wherein R^{11aa} and R^{11ba} are the same or different and each represents a hydrogen atom or lower alkyl), -CR^{11ca}R^{11da}-O-(wherein R^{11ca} and R^{11da} are the same or different and each represents a hydrogen atom or lower alkyl).

ka and ma are each preferably 1.
na is preferably 0 or 1, more preferably 0.

When R² is

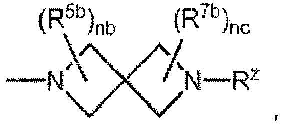

R^{Z} is aryl optionally having substituent(s) or an aromatic heterocyclic group optionally having substituent(s), more preferably aryl optionally having substituent(s), further preferably phenyl optionally having substituent(s).

R^{5b} and R^{7b} are the same or different and each is preferably halogen, hydroxy or lower alkyl optionally having substituent(s), more preferably halogen or lower alkyl optionally having substituent(s), further preferably lower alkyl optionally having substituent(s).

nb is preferably 0, and nc is preferably 0.

R³ is preferably the following (A) or (B).
(A) S(O)₂NR^{13b}R^{13c} (wherein R^{13b} and R^{13c} are each as defined above), more preferably, R^{13d} is a hydrogen atom, and R^{13c} is lower alkyl optionally having substituent(s), lower alkoxy optionally having substituent(s) or cycloalkyl optionally having substituent(s)
(B) S(O)₂NR^{13d}C(=O)R^{13e} (wherein R^{13d} and R^{13e} are each as defined above), more preferably R^{13d} is a hydrogen atom, and R^{13e} is lower alkyl optionally having substituent(s), cycloalkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s) or N,N-di-arylamino optionally having substituent(s).

R⁴ is preferably a hydrogen atom.

As compound (I), a compound, wherein one or more of the aforementioned preferable substituent embodiments are combined, is preferable.

In each group of compound (IA),

R^{1A} is preferably aryl optionally having substituent(s) or an aromatic heterocyclic group optionally having substituent(s), more preferably aryl optionally having substituent(s), further preferably phenyl optionally having substituent(s) (wherein the substituent(s) in the aryl optionally having substituent(s) or the phenyl optionally having substituent(s) is preferably lower alkyl or halogen and the number thereof is preferably 2 or 3). An aromatic heterocyclic group optionally having substituent(s) is also a more preferable embodiment.

L¹ is preferably a single bond.

When R^{2A} is

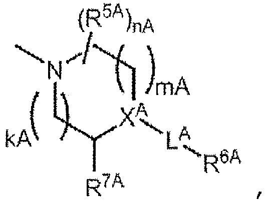

R^{5A} is preferably lower alkyl.

R^{6A} is preferably aryl optionally having substituent(s) or an aromatic heterocyclic group optionally having substituent(s), more preferably aryl optionally having substituent(s), further preferably phenyl optionally having substituent(s).

L^{A} is preferably a bond.

R^{7A} is preferably a hydrogen atom or lower alkyl.

X^{A} is preferably a nitrogen atom or -CR^{8Aa} (wherein R^{8Aa} represents a hydrogen atom, halogen or lower alkyl), more preferably -CR^{8Aa} (wherein R^{8Aa} is as defined above).

mA and kA are each preferably 1.

nA is preferably 0 or 1, more preferably 0

When R^{2A} is

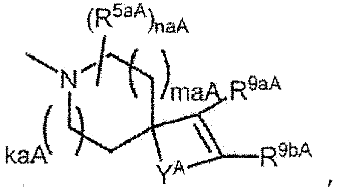

R^{5aA} is preferably lower alkyl.

kaA and maA are each preferably 1.

R^{9aA} and R^{9bA} preferably form, together with the respectively adjacent carbon atoms, a benzene ring optionally having substituent(s).

Y^{A} is preferably -CHR^{10aA}-CHR^{10bA}- (wherein R^{10aA} and R^{10bA} are each as defined above), -CR^{10cA}=CR^{10dA}- (wherein R^{10cA} and R^{10dA} are each as defined above), -O-CR^{11aA}R^{11bA}- (wherein R^{11aA} and R^{11bA} are each as defined above), or -CR^{11cA}R^{11dA}-O- (wherein R^{11cA} and R^{11dA} are each as defined above), more preferably -CHR^{10aAa}-CHR^{10bAa}- (wherein R^{10aAa} and R^{10bAa} are the same or different and each represents a hydrogen atom or lower alkyl), -CR^{10cAa}=CR^{10dA}-(wherein R^{10cAa} and R^{10dAa} are the same or different and each represents a hydrogen atom or lower alkyl), -O-CR^{11aAa}R^{11bAa}-(wherein R^{11aAa} and R^{11bAa} are the same or different and each represents a hydrogen atom or lower alkyl), or -CR^{11cAa}R^{11dAa}-O-(wherein R^{11cAa} and R^{11dAa} are the same or different and each represents a hydrogen atom or lower alkyl).

naA is preferably 0 or 1, more preferably 0.

When R^{2A} is

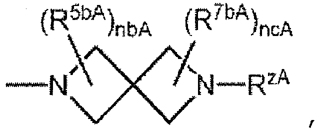

R^{zA} is preferably aryl optionally having substituent(s) or an aromatic heterocyclic group optionally having substituent(s), more preferably aryl optionally having substituent(s), further preferably phenyl optionally having substituent(s).

R^{5bA} and R^{7bA} are the same or different and each is preferably halogen, hydroxy, lower alkyl optionally having substituent(s), more preferably halogen or lower alkyl optionally having substituent(s), further preferably lower alkyl optionally having substituent(s).

nbA is preferably 0, and ncA is preferably 0.

R^{13A} is preferably a hydrogen atom or lower alkyl optionally having substituent(s), more preferably a hydrogen atom.

R^{13B} is preferably lower alkyl optionally having substituent(s), lower alkoxy optionally having substituent(s) or COR^{13e1} (wherein R^{13e1} is as defined above), more preferably lower alkyl, halogen-substituted lower alkyl (wherein the halogen moiety of the halogen-substituted lower alkyl means the same as the aforementioned halogen, lower alkyl moiety means the same as the aforementioned alkylene), or COR^{13e2} (wherein R^{13e2} represents lower alkyl optionally having substituent(s), cycloalkyl optionally having substituent(s), N-mono-lower alkylamino optionally having substituent(s) or N-mono-arylamino optionally having substituent(s), from the definition of R^{13e1}).

R^{4A} is preferably a hydrogen atom.

As compound (IA), a compound, wherein one or more of the aforementioned preferable substituent embodiments are combined, is preferable. Furthermore, compound (IA) described in (3) - (16) recited in the "Means of Solving the Problems", which is limited by preferable substituent embodiments mentioned above, is preferable. Compound (IA) described in (3) - (16) recited in the "Means of Solving the Problems", which is limited by a combination of one or more preferable substituent embodiments mentioned above, is also more preferable.

Particularly, for the ocular inflammatory diseases in the present invention, a pyrazolopyrimidine compound represented by the following formula (IB) is preferable

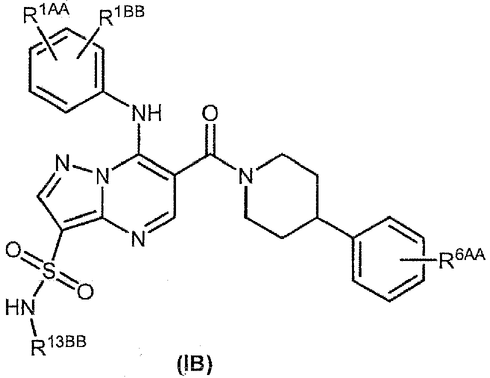

(wherein R^{1AA} and R^{1BB} are the same or different and each represents a hydrogen atom, halogen or C₁₋₁₀ alkyl, preferably fluorine atom, chlorine atom or methyl,
R^{6AA} represents a hydrogen atom or halogen, preferably a hydrogen atom or a fluorine atom, and
R^{13BB} represents C₁₋₁₀ alkyl, C₁₋₁₀ alkoxy-substituted C₁₋₁₀ alkyl, halogen-substituted C₁₋₁₀ alkyl, C₃₋₈ cycloalkyl, C₂₋₁₁ alkanoyl, C₁₋₁₀ alkoxycarbonyl, C₃₋₈ cycloalkylcarbonyl, C₁₋₁₀ alkylcarbamoyl or C₃₋₈ cycloalkylcarbamoyl, preferably C₁₋₁₀ alkylcarbamoyl or C₃₋₈ cycloalkylcarbamoyl), or a pharmaceutically acceptable salt thereof, especially, a compound wherein R^{13BB} is C₂₋₅ alkanoyl or C₁₋₄ alkylcarbamoyl is preferable.

The pharmaceutically acceptable salt of compounds (I), (IA) and (IB) includes, for example, pharmaceutically acceptable acid addition salts, metal salts, ammonium salts, organic amine addition salts, amino acid addition salts and the like. Examples of the pharmaceutically acceptable acid addition salt of compounds (I), (IA) and (IB) include inorganic acid salts such as hydrochloride, hydrobromide, nitrate, sulfate, phosphate or the like, organic acid salts such as acetate, oxalate, maleate, fumarate, citrate, benzoate, methanesulfonate or the like, or the like. Examples of the pharmaceutically acceptable metal salts include sodium salt, potassium salt, magnesium salt, calcium salt, aluminum salt, zinc salt or the like. Examples of the pharmaceutically acceptable ammonium salt include salts such as ammonium salt, tetramethylammonium salt or the like. Examples of the pharmaceutically acceptable organic amine addition salt include addition salts of morpholine, piperidine or the like. Examples of the pharmaceutically acceptable amino acid addition salt include addition salts of lysine, glycine, phenylalanine, aspartic acid, glutamic acid or the like. The pharmaceutically acceptable salts of compounds (I), (IA) and (IB) are not limited to these examples.

The ocular inflammatory diseases refer to inflammatory diseases observed in the eye. Specific examples include, but are not limited to, conjunctivitis (for example, including viral conjunctivitis, allergic conjunctival disease, bacterial conjunctivitis and the like), uveitis, keratoconjunctivitis, eyelid dermatitis, cyclitis, scleritis, episcleritis, optic neuritis, retrobulbar optic neuritis, keratitis, blepharitis, corneal ulcer, conjunctival ulcer and the like.

While the allergic conjunctival disease includes, for example, allergic conjunctivitis, atopic keratoconjunctivitis, spring catarrh, giant papillary conjunctivitis and the like, the allergic conjunctival disease in the present invention is not limited thereto.

Furthermore, while the allergic conjunctivitis includes, for example, seasonal allergic conjunctivitis such as pollinosis and the like, perennial allergic conjunctivitis and the like, the allergic conjunctivitis in the present invention is not limited thereto.

Compounds (I), (IA) and (IB) used in the present invention also encompasses a prodrug thereof. The prodrug of compound (I), (IA) and (IB) is a compound that is converted to compound (I), (IA) or (IB) by a reaction with enzyme, gastric acid and the like in the body. Many kinds of prodrugs are known, and can be synthesized by selecting a suitable prodrug based on a known literature (Iyakuhin no Kaihatsu, Hirokawa Shoten, 1990, vol.7, p.163) and using a known method. As a prodrug of compound (I), (IA) and (IB), for example, when compound (I), (IA) and (IB) have an amino group, compounds wherein the amino group is acylated, alkylated or phosphorylated can be mentioned; when compound (I), (IA) and (IB) have a hydroxy group, compounds wherein the hydroxy group is acylated, alkylated, phosphorylated or borated can be mentioned; when compound (I), (IA) and (IB) have a carboxyl group, compounds wherein the carboxyl group is esterified or amidated and the like can be mentioned. Also, the prodrug of compound (I), (IA) and (IB) may be any of hydrate, non-hydrate and solvate, and may form a salt with a pharmaceutically acceptable acid or a base, in the same manner as in the case of compounds (I), (IA) and (IB).

Some of the compounds (I), (IA) and (IB) used in the present invention can exist as a stereoisomer such as geometric isomer, optical isomer and the like, tautomer and the like. The compound to be used in the present invention encompasses all possible isomers and mixtures thereof including these, and the mixing ratio thereof may be any ratio.

Compounds (I), (IA) and (IB), and pharmaceutically acceptable salts thereof to be used in the present invention may be present as adducts with water or various solvents. Such adducts are also used in the present invention.

The atoms in the compounds (I), (IA) and (IB) used in the present invention may be partially or entirely replaced by the respective corresponding isotope atom(s), and such compounds replaced by isotope atom(s) can be used in the present invention. For example, hydrogen atoms in compound (I), (IA) and (IB) may be a hydrogen atom having an atomic weight of 2 (deuterium atom).

A compound wherein the atoms in the compounds (I), (IA) and (IB) are partially or entirely replaced by the respective corresponding isotope atom(s) can be produced using a commercially available building block and in the same manner as in each production method described in, for example, WO 2011/108689.

Compounds (I), (IA) and (IB) can be produced in the same manner as in each production method described in, for example, WO 2011/108689, WO 2009/041663.

Specific examples of the compounds (I), (IA) and (IB) used in the present invention are shown in Tables 1 - 13. However, the compounds to be used in the present invention are not limited to them.

**[Table 1]**

| | | | | |
|---|---|---|---|---|
| Table 1 | | 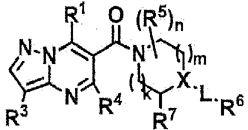 | | |

| Compound No. | 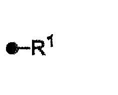 | 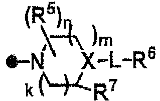 | 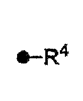 | 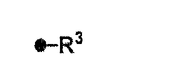 |
|---|---|---|---|---|
| a-1 | 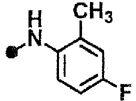 | 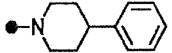 | 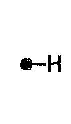 | 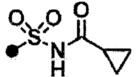 |
| a-2 | 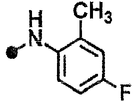 | 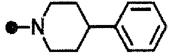 | 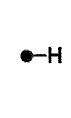 | 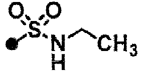 |
| a-3 | 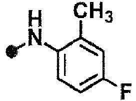 | 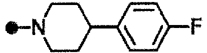 | 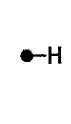 | 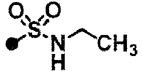 |
| a-4 | 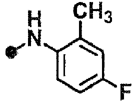 | 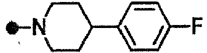 | 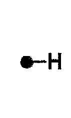 | 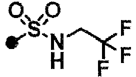 |
| a-5 | 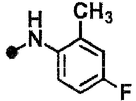 | 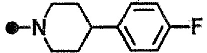 | 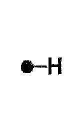 | 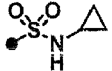 |
| a-6 | 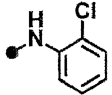 | 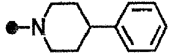 | 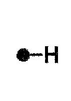 | 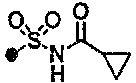 |
| a-7 | 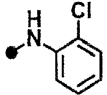 | 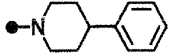 | 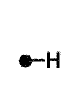 | 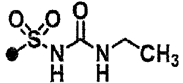 |
| a-8 | 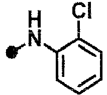 | 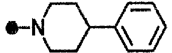 | 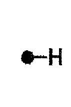 | 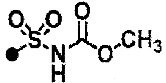 |
| a-9 | 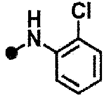 | 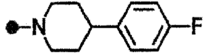 | 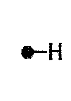 | 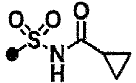 |
| a-10 | 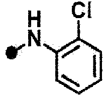 | 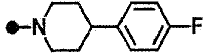 | 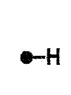 | 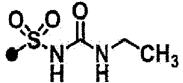 |

**[Table 2]**

| | | | | |
|---|---|---|---|---|
| Table 2 | | 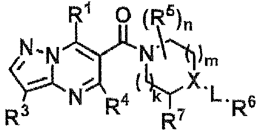 | | |

| Compound No. | 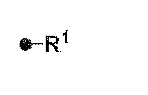 | 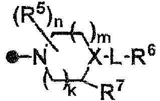 | 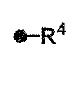 | 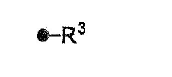 |
|---|---|---|---|---|
| **a-11** | 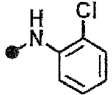 | 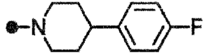 | 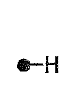 | 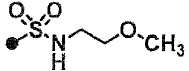 |
| **a-12** | 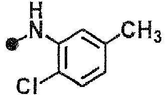 | 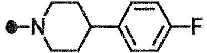 | 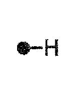 | 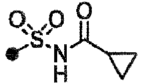 |
| **a-13** | 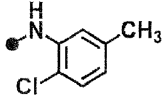 | 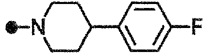 | 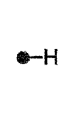 | 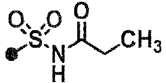 |
| **a-14** | 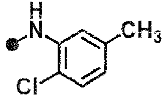 | 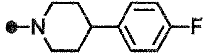 | 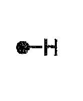 | 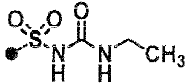 |
| **a-15** | 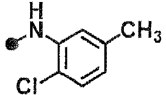 | 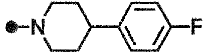 | 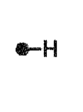 | 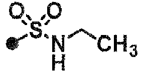 |
| **a-16** | 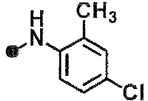 | 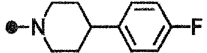 | 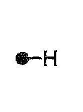 | 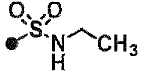 |
| **a-17** | 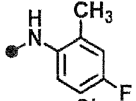 | 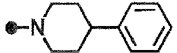 | 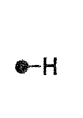 | 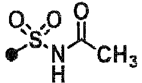 |
| **a-18** | 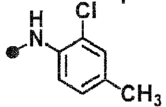 | 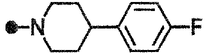 | 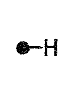 | 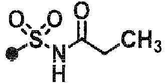 |
| **a-19** | 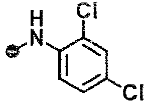 | 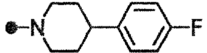 | 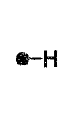 | 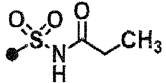 |
| **a-20** | 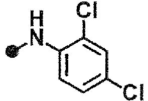 | 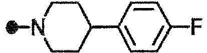 | 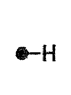 | 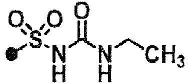 |

**[Table 3]**

| | | | | |
|---|---|---|---|---|
| Table 3 | | 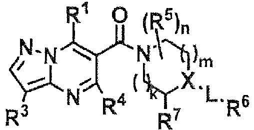 | | |

| Compound No. | 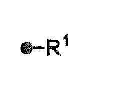 | 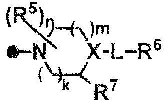 | 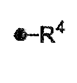 | 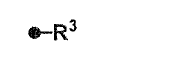 |
|---|---|---|---|---|
| **a-21** | 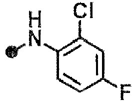 | 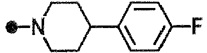 | 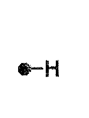 | 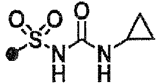 |
| **a-22** | 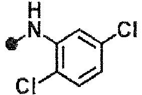 | 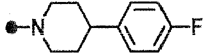 | 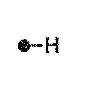 | 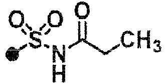 |
| **a-23** | 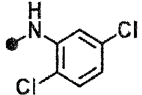 | 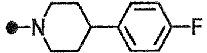 | 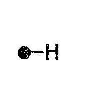 | 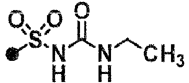 |
| **a-24** | 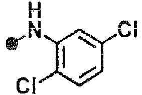 | 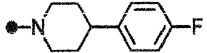 | 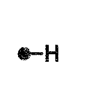 | 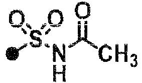 |
| **a-25** | 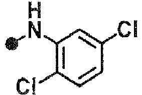 | 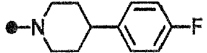 | 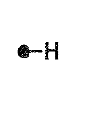 | 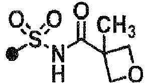 |
| **a-26** | 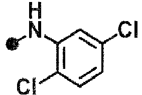 | 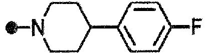 | 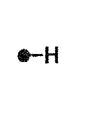 | 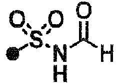 |
| **a-27** | 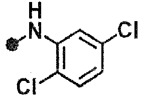 | 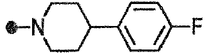 | 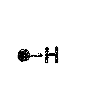 | 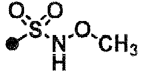 |
| **a-28** | 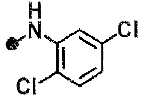 | 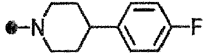 | 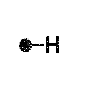 | 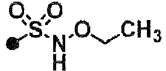 |
| **a-29** | 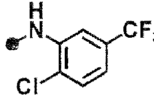 | 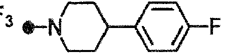 | 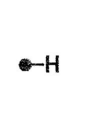 | 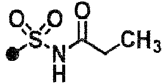 |
| **a-30** | 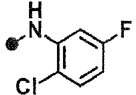 | 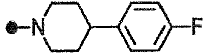 | 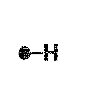 | 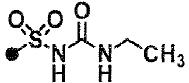 |

**[Table 4]**

| | | | | |
|---|---|---|---|---|
| Table 4 | | 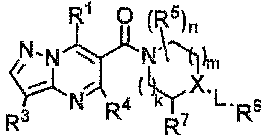 | | |

| Compound No. | 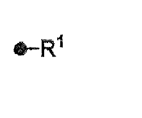 | 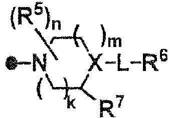 | 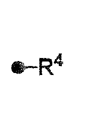 | 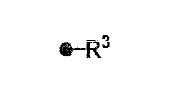 |
|---|---|---|---|---|
| **a**-**31** | 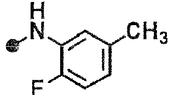 | 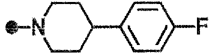 | 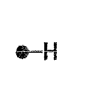 | 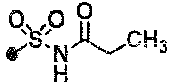 |
| **a**-**32** | 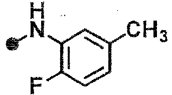 | 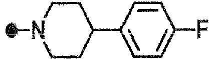 | 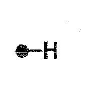 | 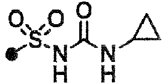 |
| **a**-**33** | 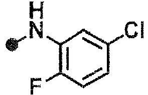 | 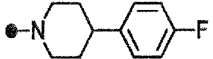 | 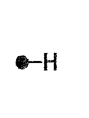 | 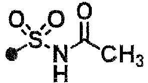 |
| **a**-**34** | 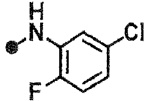 | 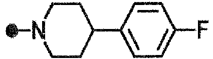 | 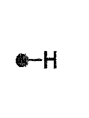 | 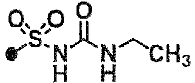 |
| **a**-**35** | 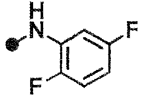 | 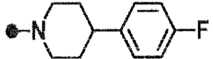 | 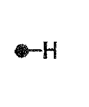 | 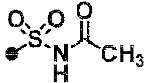 |
| **a**-**36** | 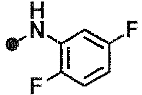 | 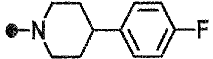 | 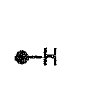 | 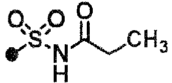 |
| **a**-**37** | 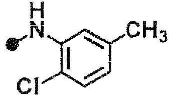 | 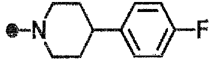 | 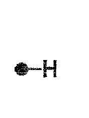 | 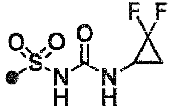 |
| **a**-**38** | 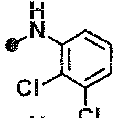 | 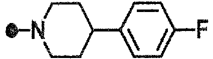 | 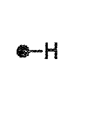 | 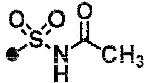 |
| **a**-**39** | 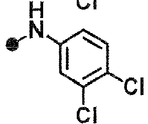 | 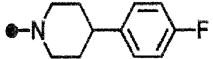 | 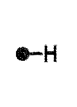 | 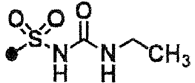 |

**[Table 5]**

| | | | | |
|---|---|---|---|---|
| Table 5 | | 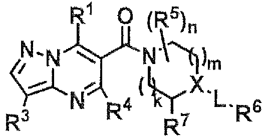 | | |

| Compound No. | 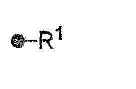 | 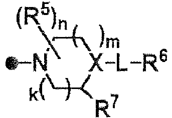 | 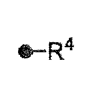 | 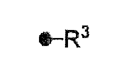 |
|---|---|---|---|---|
| **a**-**40** | 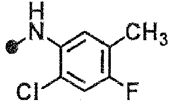 | 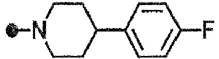 | 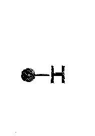 | 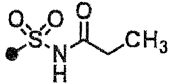 |
| **a**-**41** | 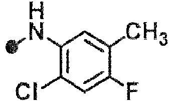 | 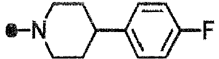 | 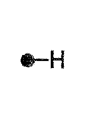 | 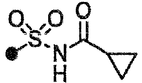 |
| **a**-**42** | 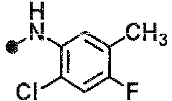 | 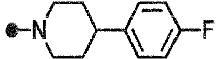 | 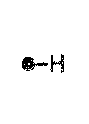 | 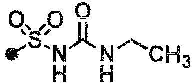 |

**[Table 6]**

| | | | | |
|---|---|---|---|---|
| Table 6 | | 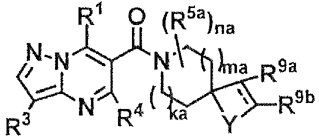 | | |

| Compound No. | 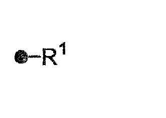 | 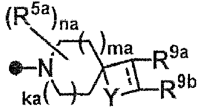 | 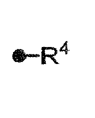 | 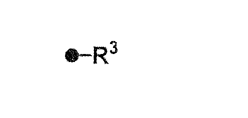 |
|---|---|---|---|---|
| **a-43** | 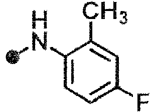 | 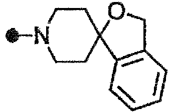 | 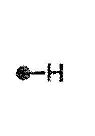 | 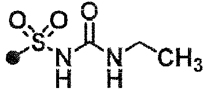 |
| **a-44** | 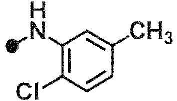 | 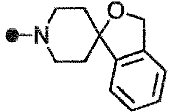 | 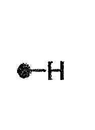 | 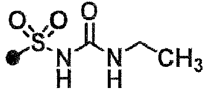 |
| **a-45** | 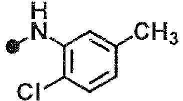 | 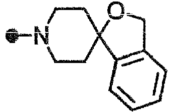 | 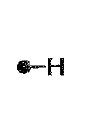 | 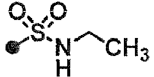 |
| **a-46** | 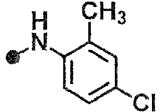 | 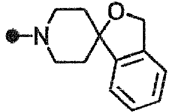 | 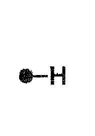 | 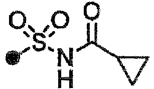 |
| **a-47** | 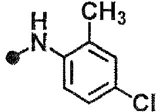 | 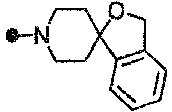 | 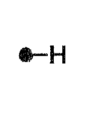 | 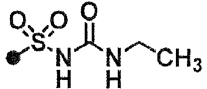 |
| **a-48** | 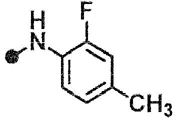 | 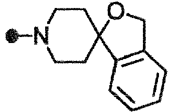 | 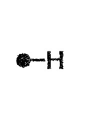 | 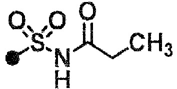 |
| **a-49** | 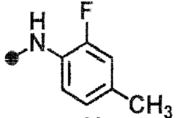 | 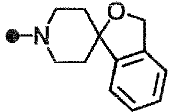 | 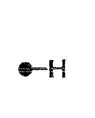 | 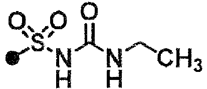 |
| **a-50** | 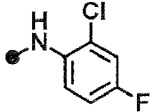 | 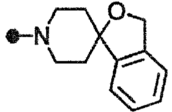 | 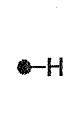 | 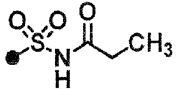 |

**[Table 7]**

| | | | | |
|---|---|---|---|---|
| Table 7 | | 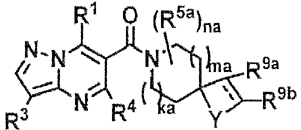 | | |

| Compound No. | 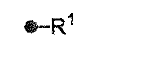 | 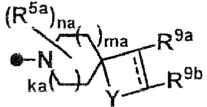 | 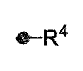 | 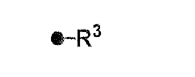 |
|---|---|---|---|---|
| **a**-**51** | 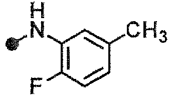 | 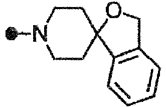 | 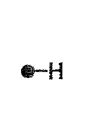 | 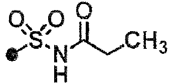 |
| **a**-**52** | 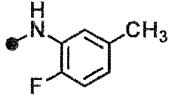 | 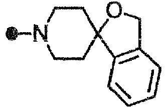 | 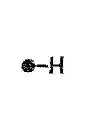 | 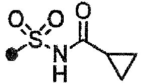 |
| **a**-**53** | 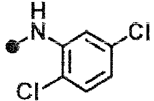 | 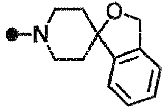 | 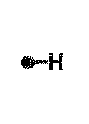 | 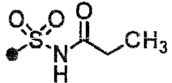 |
| **a**-**54** | 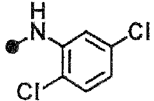 | 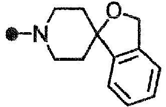 | 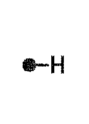 | 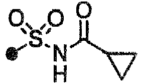 |
| **a**-**55** | 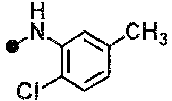 | 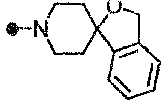 | 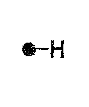 | 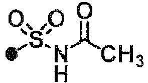 |
| **a**-**56** | 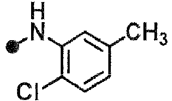 | 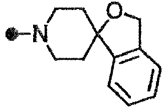 | 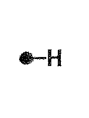 | 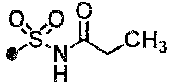 |
| **a**-**57** | 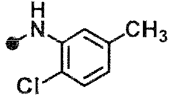 | 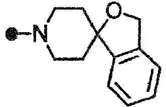 | 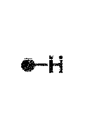 | 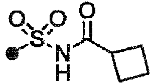 |
| **a**-**58** | 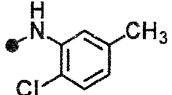 | 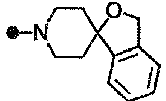 | 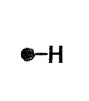 | 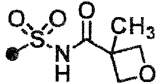 |
| **a**-**59** | 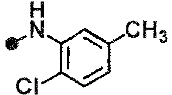 | 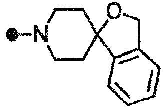 | 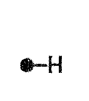 | 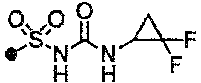 |

| | | | | |
|---|---|---|---|---|
| [Table 8] | | | | |
| Table 8 | 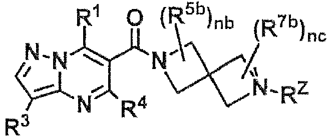 | | | |

| Compound No. | 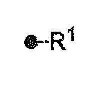 | 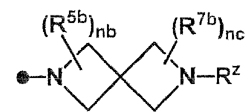 | 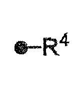 | 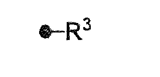 |
|---|---|---|---|---|
| **a-60** | 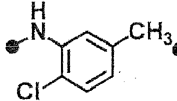 | 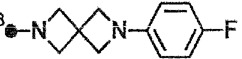 | 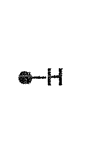 | 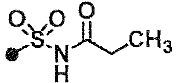 |
| **a**-**61** | 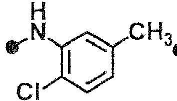 | 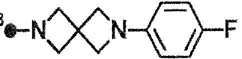 | 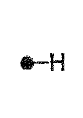 | 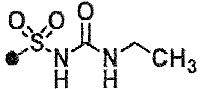 |

**[Table 9]**

| | | | |
|---|---|---|---|
| Table 9 | 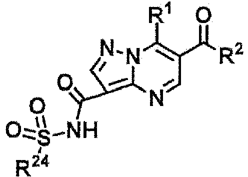 | | |

| Compound No. | 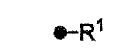 | 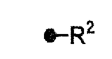 | 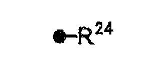 |
|---|---|---|---|
| **a**-**62** | 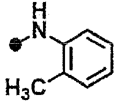 | 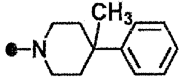 | 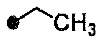 |
| **a**-**63** | 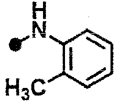 | 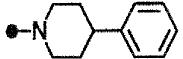 | 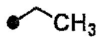 |
| **a-64** | 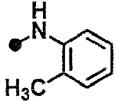 | 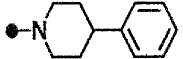 | 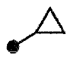 |
| **a**-**65** | 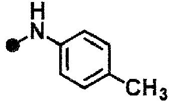 | 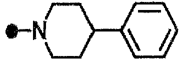 | 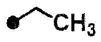 |
| **a**-**66** | 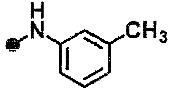 | 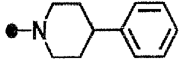 | 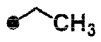 |
| **a**-**67** | 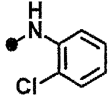 | 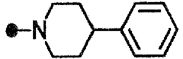 | 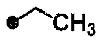 |
| **a**-**68** | 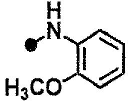 | 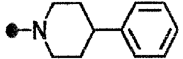 | 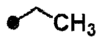 |
| **a**-**69** | 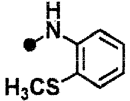 | 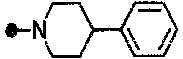 | 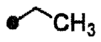 |
| **a-70** | 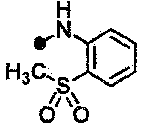 | 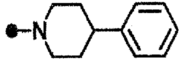 | 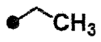 |

[Table 10]

**Table 10 (continued from Table 9)**

| Compound No. | 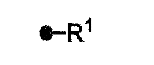 | 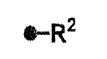 | 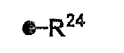 |
|---|---|---|---|
| **a**-**71** | 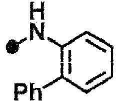 | 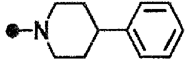 | 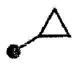 |
| **a**-**72** | 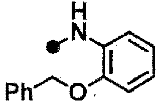 | 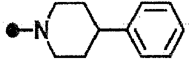 | 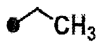 |
| **a**-**73** | 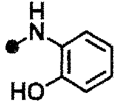 | 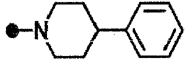 | 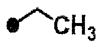 |
| **a**-**74** | 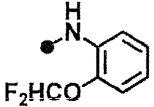 | 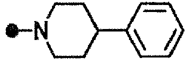 | 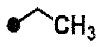 |
| **a-75** | 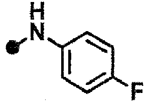 | 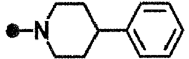 | 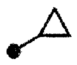 |
| **a**-**76** | 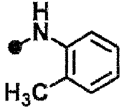 | 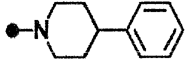 | 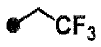 |
| **a**-**77** | 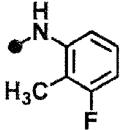 | 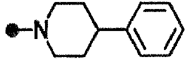 | 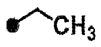 |
| **a**-**78** | 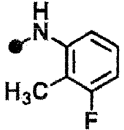 | 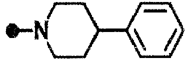 | 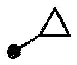 |
| **a**-**79** | 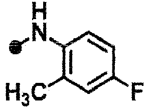 | 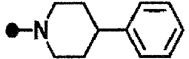 | 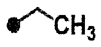 |
| **a**-**80** | 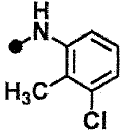 | 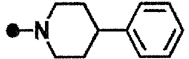 | 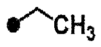 |

[Table 11]

**Table 11 (continued from Table 9)**

| Compound No. | ●-R¹ | ●-R² | ●-R²⁴ |
|---|---|---|---|
| **a-81** | 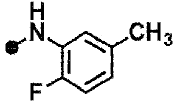 | 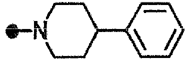 | 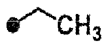 |
| **a**-**82** | 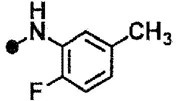 | 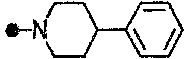 | 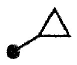 |
| **a**-**83** | 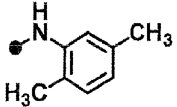 | 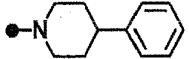 | 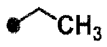 |
| **a**-**84** | 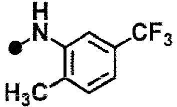 | 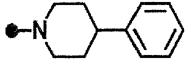 | 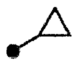 |
| **a**-**85** | 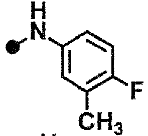 | 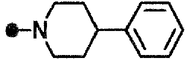 | 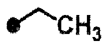 |
| **a**-**86** | 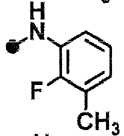 | 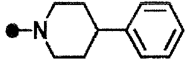 | 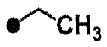 |
| **a**-**87** | 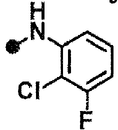 | 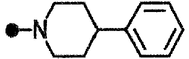 | 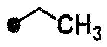 |
| **a**-**88** | 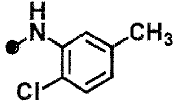 | 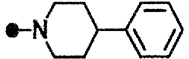 | 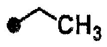 |
| **a-89** | 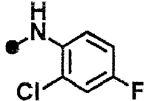 | 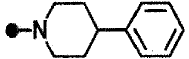 | 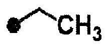 |
| **a**-**90** | 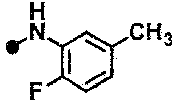 | 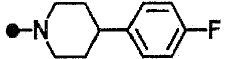 | 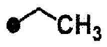 |

[Table 12]

**Table 12 (continued from Table 9)**

| Compound No. | ●**-R¹** | ●**-R²** | ●-R**²⁴** |
|---|---|---|---|
| **a**-**91** | 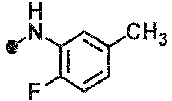 | 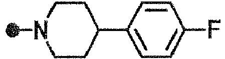 | 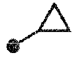 |
| **a**-**92** | 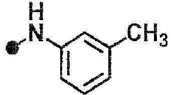 | 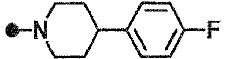 | 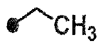 |
| **a**-**93** | 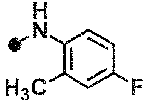 | 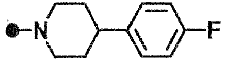 | 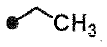 |
| **a**-**94** | 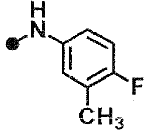 | 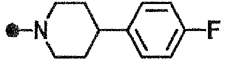 | 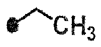 |
| **a-95** | 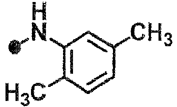 | 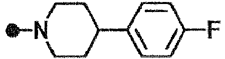 | 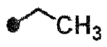 |
| **a**-**96** | 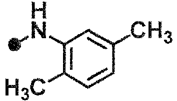 | 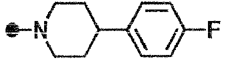 | 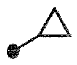 |
| **a**-**97** | 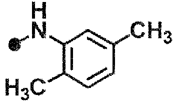 | 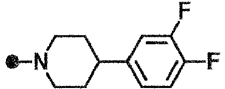 | 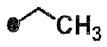 |
| **a**-**98** | 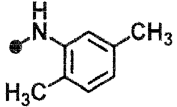 | 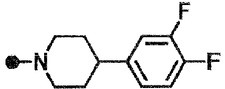 | 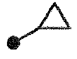 |
| **a**-**99** | 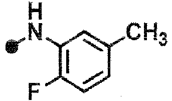 | 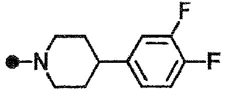 | 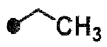 |
| **a**-**100** | 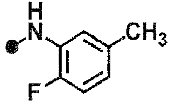 | 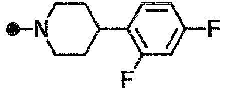 | 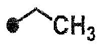 |

[Table 13]

**Table 13 (continued from Table 9)**

| Compound No. | ●**-R¹** | ●**-R²** | ●**-R²⁴** |
|---|---|---|---|
| **a**-**101** | 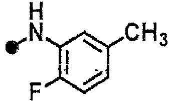 | 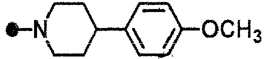 | 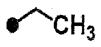 |
| **a-102** | 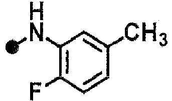 | 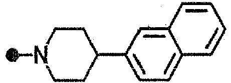 | 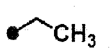 |
| **a**-**103** | 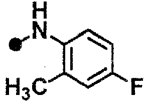 | 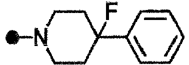 | 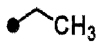 |
| **a-104** | 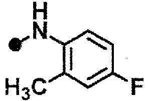 | 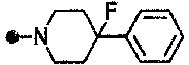 | 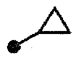 |
| **a**-**105** | 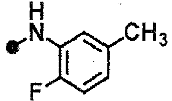 | 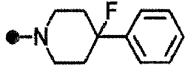 | 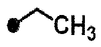 |
| **a**-**106** | 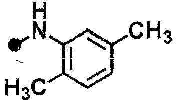 | 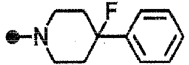 | 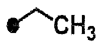 |
| **a-107** | 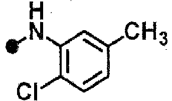 | 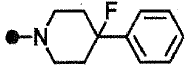 | 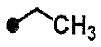 |
| **a-108** | 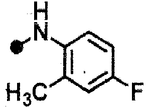 | 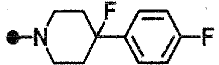 | 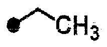 |
| **a**-**109** | 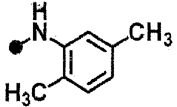 | 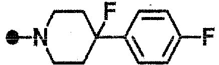 | 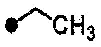 |

Next, the pharmacological effect of the representative compounds is specifically explained by Experimental Examples. [Experimental Example 1] Effect on late phase reaction in mouse model of ragweed pollen-induced conjunctivitis

To examine effect of the compound on ocular inflammatory diseases, a mouse model of ragweed pollen-induced conjunctivitis which is an animal model of allergic conjunctivitis pathology [Allergy, 2003, vol.58, pages 1101 - 1013] was used. Eosinophil infiltration into conjunctiva was evaluated as an index of a late phase reaction of the conjunctivitis model. The suppressive effect on eosinophil infiltration was examined by administering compound a-14 orally or ophthalmically. Compound a-14 used in this test was synthesized by reference to the method described in Example 14 of WO 2011/108689.

Male BALB/c mice (CLEA Japan, Inc., 6-week-old) were stocked, and conjunctivitis models were prepared using individuals that showed no abnormality after quarantine and acclimation for 1 week.

One group contained 10 animals, and 3 groups of a control group (oral administration and ophthalmic administration of medium), an a-14 oral administration group (oral administration of a-14, ophthalmic administration of medium), and an a-14 ophthalmic administration group (oral administration of medium, ophthalmic administration of a-14) were set.

An antigen solution for sensitization was prepared by adding ragweed pollen (Polysciences, Inc., 10 mg) to aluminum hydroxide gel (13 mg/mL, Sigma-Aldrich, 10 mL), and stirring the mixture for not less than 1 hr (ragweed pollen concentration: 1 mg/mL). An eliciting antigen solution was prepared by adding ragweed pollen (100 mg) to phosphate buffered saline (PBS) (Life Technologies Corporation, 1 mL) (ragweed pollen concentration 100 mg/mL).

The initial sensitization date being day 0, the antigen solution for sensitization was subcutaneously administered to the foot plantar of both feet of the mouse by 100 µL each on day 0. For booster, the antigen solution for sensitization (200 µL) was intraperitoneally administered to the mouse abdomen on day 8. On day 18, the eliciting antigen solution was ophthalmically administered to the both eyes (5 µL for each eye) of the mouse to perform the first elicitation. On day 32, the eliciting antigen solution was ophthalmically administered to the both eyes (5 µL for each eye) of the mouse to perform the second elicitation. Grouping was performed based on the body weight on day 8.

Methylcellulose 400 (Wako Pure Chemical Industries, Ltd.) was measured, and dissolved in the Japanese Pharmacopoeia, water for injection (Otsuka Pharmaceutical Factory, Inc.) to a concentration of 0.5 w/v% to give 0.5 w/v% methylcellulose 400 solution, which was used as an oral medium. Compound a-14 was suspended in the oral medium to a concentration of 1 mg/mL to give a compound liquid for oral administration.

PBS was used as an ophthalmic medium. Compound a-14 was suspended in the ophthalmic medium to a concentration of 1 mg/mL to prepare a compound liquid for ophthalmic administration.

Oral administration was performed for 17 days from before the first elicitation on day 17 to after the second elicitation on day 33, twice every day at 7:00 - 7:59 and 18:00 - 18:59. However, it was performed only once on day 33 at 7:00 - 7:59. The compound liquid for oral administration or oral medium was orally administered using a 1 mL syringe and a stomach tube at 0.3 mL per mouse.

Ophthalmic administration was performed for 6 days on days 17, 18, 19, 31, 32 and 33. The administration was performed 5 times per day on one day before elicitation (days 17 and 31) and the elicitation day (days 18 and 32), in the time periods of 7:00 - 7:59, 10:00 - 10:59, 13:00 - 13:59, 16:00 - 16:59 and 19:00 - 19:59 of one day before elicitation (days 17 and 31) and 7:00 - 7:59, 9:00 - 9:59, 11:00 - 11:59, 13:00 - 13:59 and 16:00 - 16:59 of the elicitation day (days 18 and 32). The administration was performed only once in 7:00 - 7:59 of one day after the first and the second elicitation (days 19 and 33). The compound liquid for ophthalmic administration or ophthalmic medium was instilled to the both eyes by 5 µL each eye by using a micropipette (10 µL per mouse).

About 24 hr after the second elicitation on day 32, all individuals were laparotomized under sodium pentobarbital anesthesia (about 50 mg/kg, intraperitoneal administration), and allowed to die from exsanguination. After confirmation of cardiac arrest, they were decapitated and the head was preserved in 10% phosphate buffered formalin solution (Wako Pure Chemical Industries, Ltd.). The decapitated head was decalcified including the skull and embedded in paraffin, which was sliced to give serial sections.

The sections were Congo red stained, and the number of eosinophils in the upper and lower palpebral conjunctiva (per individual specimen) of the both eyes was counted under an optical microscope. The effect of the compound on eosinophil infiltration was evaluated based on of the number of eosinophils per individual specimen.

The results of eosinophil cell count are shown in Table 14.
[Table 14]

**Table 14**

| Group constitution | dose | n | number of eosinophils |
|---|---|---|---|
| Control group | - | 10 | 95.5 ± 15.5 |
| a-14 oral administration group | 0.3 mg/body | 10 | 64.8 ± 6.2 |
| a-14 ophthalmic administration group | 10 µg/eyes | 10 | 70.4 ± 16.4 |

The number of eosinophils of each group is shown in mean±standard error.

### [Experimental Example 2] Effect on late phase reaction in mouse model of ragweed pollen-induced conjunctivitis

To examine effect of the compound on ocular inflammatory diseases, a mouse model of ragweed pollen-induced conjunctivitis which is an animal model of allergic conjunctivitis pathology [Allergy, 2003, vol.58, pages 1101 - 1013] was used. Eosinophil infiltration into conjunctiva was evaluated as an index of a late phase reaction of the conjunctivitis model. The suppressive action on eosinophil infiltration was examined by administering the compound orally or ophthalmically. Compounds a-22, a-27, a-33, a-34, a-35 and a-44 used in this test were each synthesized by reference to the methods described in Examples 22, 27, 33, 34, 35 and 44 of WO 2011/108689, and compounds a-79, a-90 and a-93 used in this test were each synthesized by reference to the methods described in Examples 57, 81 and 84 of WO 2009/041663.

Male BALB/c mice (CLEA Japan, Inc., 6-week-old) were stocked, and conjunctivitis models were prepared using individuals that showed no abnormality after quarantine and acclimation for 1 week.

One group contained 10 animals, and groups of a control group (oral administration and ophthalmic administration of medium), a compound oral administration group (oral administration of compound, ophthalmic administration of medium), and a compound ophthalmic administration group (oral administration of medium, ophthalmic administration of compound) were set.

An antigen solution for sensitization was prepared by adding ragweed pollen (Polysciences, Inc., 10 mg) to aluminum hydroxide gel (13 mg/mL, Sigma-Aldrich, 10 mL), and stirring the mixture for not less than 1 hr (ragweed pollen concentration: 1 mg/mL). An eliciting antigen solution was prepared by adding ragweed pollen (100 mg) to phosphate buffered saline (PBS) (Life Technologies Corporation, 1 mL) (ragweed pollen concentration 100 mg/mL).

The initial sensitization date being day 0, the antigen solution for sensitization was subcutaneously administered to the foot plantar of both feet of the mouse by 100 µL each on day 0. For booster, the antigen solution for sensitization (200 µL) was intraperitoneally administered to the mouse abdomen on day 8. On day 18, the eliciting antigen solution was ophthalmically administered to the both eyes (5 µL for each eye) of the mouse to perform the first elicitation. On day 32, the eliciting antigen solution was ophthalmically administered to the both eyes (5 µL for each eye) of the mouse to perform the second elicitation. Grouping was performed based on the body weight on day 8.

Methylcellulose 400 (Wako Pure Chemical Industries, Ltd.) was measured, and dissolved in the Japanese Pharmacopoeia, water for injection (Otsuka Pharmaceutical Factory, Inc.) to a concentration of 0.5 w/v% to give 0.5 w/v% methylcellulose 400 solution, which was used as an oral medium. The compound was suspended in the oral medium to a concentration of 1 mg/mL to give a compound liquid for oral administration.

PBS containing 5 w/v% dimethyl sulfoxide (DMSO) (Wako Pure Chemical Industries, Ltd.) was used as an ophthalmic medium. The compound was suspended in the ophthalmic medium to a concentration of 1 mg/mL to prepare a compound liquid for ophthalmic administration.

Oral administration was performed for 17 days from before the first elicitation on day 17 to after the second elicitation on day 33, twice every day at 7:00 - 7:59 and 18:00 - 18:59. However, it was performed only once on day 33 at 7:00 - 7:59. The compound liquid for oral administration or oral medium was orally administered using a 1 mL syringe and a stomach tube at 0.3 mL per mouse.

Ophthalmic administration was performed for 6 days on days 17, 18, 19, 31, 32 and 33. The administration was performed 5 times per day on one day before elicitation (days 17 and 31) and the elicitation day (days 18 and 32), in the time periods of 7:00 - 7:59, 10:00 - 10:59, 13:00 - 13:59, 16:00 - 16:59 and 19:00 - 19:59 of one day before elicitation (days 17 and 31) and 7:00 - 7:59, 9:00 - 9:59, 11:00 - 11:59, 13:00 - 13:59 and 16:00 - 16:59 of the elicitation day (days 18 and 32). The administration was performed only once in 7:00 - 7:59 of one day after the first and the second elicitation (says 19 and 33). The ophthalmic administration compound liquid or Ophthalmic medium was instilled to the both eyes by 5 µL each eye by using a micropipette (10 µL per mouse).

About 24 - 27 hr after the second elicitation on day 32, all individuals were laparotomized under sodium pentobarbital anesthesia (about 50 mg/kg, intraperitoneal administration), and allowed to die from exsanguination. After confirmation of cardiac arrest, they were decapitated and the head was preserved in 10% phosphate buffered formalin solution (Wako Pure Chemical Industries, Ltd.). The decapitated head was decalcified including the skull and embedded in paraffin, which was sliced to give serial sections.

The sections were Congo red stained, and the number of eosinophils in the upper and lower palpebral conjunctiva (per individual specimen) of the both eyes was counted under an optical microscope. The effect of the compound on eosinophil infiltration was evaluated based on of the number of eosinophils per individual specimen.

The results of eosinophil cell counts are shown in Table 15.
[Table 15]

**Table 15**

| Group constitution | Dose | population | number of eosinophils |
|---|---|---|---|
| Control group | - | 10 | 205 ± 39 |
| a-22 oral administration group | 0.3 mg/body | 10 | 53 ± 12 |
| a-22 ophthalmic administration group | 10 µg/eyes | 10 | 64 ± 21 |
| a-27 oral administration group | 0.3 mg/body | 10 | 39 ± 5 |
| a-33 ophthalmic administration group | 10 µg/eyes | 10 | 83 ± 19 |
| a-34 ophthalmic administration group | 10 µg/eyes | 10 | 50 ± 10 |
| a-35 ophthalmic administration group | 10 µg/eyes | 10 | 84 ± 18 |
| a-44 oral administration group | 0.3 mg/body | 10 | 75 ± 24 |
| a-79 ophthalmic administration group | 10 µg/eyes | 10 | 52 ± 11 |
| a-90 oral administration group | 0.3 mg/body | 10 | 44 ± 7 |
| a-93 oral administration group | 0.3 mg/body | 10 | 35 ± 8 |

The number of eosinophils of each group is shown in mean ± standard error.

From the above test results, the above-mentioned test compounds showed a suppressive effect on eosinophil infiltration into the conjunctiva by both the oral administration and the ophthalmic administration.

The eosinophil infiltration or the concentration of eosinophil basic protein is known to correlate with the disease activity of allergic conjunctivitis (for example, seasonal allergic conjunctivitis such as pollinosis and the like, perennial allergic conjunctivitis and the like), spring catarrh, atopic conjunctivitis, or giant papillary conjunctivitis [British Journal of Ophthalmology, 1966, vol. 80, pages 556 - 560] [British Journal of Ophthalmology, 2004, vol.88, pages 1504 - 1505].

From the above, compounds (I), (IA) and (IB) are suggested to be useful as a prophylactic and/or therapeutic agent for ocular inflammatory diseases such as eyelid dermatitis, keratoconjunctivitis, scleritis, conjunctivitis {for example, allergic conjunctival diseases [for example, atopic keratoconjunctivitis, spring catarrh, giant papillary conjunctivitis, allergic conjunctivitis (for example, seasonal allergic conjunctivitis such as pollinosis and the like, perennial allergic conjunctivitis and the like) and the like] and the like} and the like.

While compounds (I), (IA) and (IB), and pharmaceutically acceptable salts thereof to be used in the present invention can be directly administered singly, generally, they are desirably provided as various pharmaceutical preparations. Also, such pharmaceutical preparations are used for animals and humans.

The pharmaceutical preparation of the present invention can contain, as an active ingredient, compound (I), (IA) or (IB), or a pharmaceutically acceptable salt thereof singly or as a mixture with any other active ingredient for treatment. Also, such pharmaceutical preparation is produced by any method well known in the technical field of drug formulation study, by mixing the active ingredient with one or more kinds of pharmaceutically acceptable carriers (for example, diluent, solvent, excipient and the like).

As the administration route, one most effective for the treatment is desirably employed, which may be oral or parenteral such as intravenous administration, instillation or the like.

Examples of the administration form include tablet, injection, eye drop and the like.

Tablet and the like which are suitable for oral administration can be produced using excipients such as lactose and the like, disintegrants such as starch and the like, lubricants such as magnesium stearate and the like, binders such as hydroxypropylcellulose and the like, and the like.

Injection and the like which are suitable for parenteral administration can be produced using diluent, solvent and the like, such as a salt solution, a glucose solution, a mixture of saline and glucose solution, and the like.

Eye drop can be produced using buffering agents such as phosphate buffer, borate buffer and the like, isotonic agents such as sodium chloride and the like, preservatives such as benzalkonium chloride, p-hydroxybenzoic acid ester and the like, and the like.

The present invention is explained in more detail in the following by referring to Examples, which are not to be construed as limitative.

### Example 1

### Formulation Example 1 (tablet)

A tablet having the following composition is prepared by a conventional method. Compound a-14 (40 g), lactose (286.8 g) and potato starch (60 g) are mixed, and a 10% aqueous hydroxypropylcellulose solution (120 g) is added thereto. The mixture is kneaded, granulated, dried, and sieved to give granules for tableting by a conventional method. Magnesium stearate (1.2 g) is added and mixed, and the mixture is tableted by a tableting machine (RT-15 manufactured by Kikusui Seisakusho Ltd.) with a 8 mm diameter punch to give a tablet (containing 20 mg of active ingredient per tablet).

**[Table 16]**

| Formulation | | |
|---|---|---|
| | compound a-14 | 20 mg |
| | lactose | 143.4 mg |
| | potato starch | 30 mg |
| | hydroxypropylcellulose | 6 mg |
| | magnesium stearate | 0.6 mg |
| | | 200 mg |

### Example 2

### Formulation Example 2 (injection)

An injection having the following composition is prepared by a conventional method. Compound a-14 (1 g) and D-mannitol (5 g) are added to distilled water for injection and mixed. Hydrochloric acid and aqueous sodium hydroxide solution are further added to adjust the mixture to pH 6, and distilled water for injection is added to the total amount of 1000 mL. The obtained mixture is aseptically filled in a glass vial by 2 mL to give an injection (containing 2 mg of active ingredient per vial).

**[Table 17]**

| Formulation | | |
|---|---|---|
| | compound a-14 | 2 mg |
| | D-mannitol | 10 mg |
| | hydrochloric acid | q.s. |
| | aqueous sodium hydroxide solution | q.s. |
| | distilled water for injection | q.s. |
| | | 2.00 mL |

### Example 3

### Formulation Example 3 (eye drop)

An eye drop having the following composition is prepared by a conventional method. Compound a-14 (1 g) and sodium chloride (9 g) are added to distilled water for injection and mixed. Hydrochloric acid and aqueous sodium hydroxide solution are further added to adjust the mixture to pH 7, and distilled water for injection is added to the total amount of 1000 mL. The obtained mixture is aseptically filled in an instillation container by 1 mL to give an eye drop (containing 1 mg of active ingredient per container).

**[Table 18]**

| Formulation | | |
|---|---|---|
| | compound a-14 | 1 mg |
| | sodium chloride | 9 mg |
| | hydrochloric acid | q.s. |
| | aqueous sodium hydroxide solution | q.s. |
| | distilled water for injection | q.s. |
| | | 1.00 mL |

### Example 4

### Formulation Example 4 (tablet)

A tablet having the following composition is prepared by a conventional method. Compound a-22 (40 g), lactose (286.8 g) and potato starch (60 g) are mixed, and a 10% aqueous hydroxypropylcellulose solution (120 g) is added thereto. The mixture is kneaded, granulated, dried, and sieved to give granules for tableting by a conventional method. Magnesium stearate (1.2 g) is added and mixed, and the mixture is tableted by a tableting machine (RT-15 manufactured by Kikusui Seisakusho Ltd.) with a 8 mm diameter punch to give a tablet (containing 20 mg of active ingredient per tablet).

**[Table 19]**

| Formulation | |
|---|---|
| compound a-22 | 20 mg |
| lactose | 143.4 mg |
| potato starch | 30 mg |
| hydroxypropylcellulose | 6 mg |
| magnesium stearate | 0.6 mg |
| | 200 mg |

### Example 5

### Formulation Example 5 (injection)

An injection having the following composition is prepared by a conventional method. Compound a-22 (1 g) and D-mannitol (5 g) are added to distilled water for injection and mixed. Hydrochloric acid and aqueous sodium hydroxide solution are further added to adjust the mixture to pH 6, and distilled water for injection is added to the total amount of 1000 mL. The obtained mixture is aseptically filled in a glass vial by 2 mL to give an injection (containing 2 mg of active ingredient per vial).

**[Table 20]**

| Formulation | | |
|---|---|---|
| | compound a-22 | 2 mg |
| | D-mannitol | 10 mg |
| | hydrochloric acid | q.s. |
| | aqueous sodium hydroxide solution | q.s. |
| | distilled water for injection | q. s. |
| | | 2.00 mL |

### Example 6

### Formulation Example 6 (eye drop)

An eye drop having the following composition is prepared by a conventional method. Compound a-22 (1 g) and sodium chloride (9 g) are added to distilled water for injection and mixed. Hydrochloric acid and aqueous sodium hydroxide solution are further added to adjust the mixture to pH 7, and distilled water for injection is added to the total amount of 1000 mL. The obtained mixture is aseptically filled in an instillation container by 1 mL to give an eye drop (containing 1 mg of active ingredient per container).

**[Table 21]**

| Formulation | | |
|---|---|---|
| | compound a-22 | 1 mg |
| | sodium chloride | 9 mg |
| | hydrochloric acid | q.s. |
| | aqueous sodium hydroxide solution | q.s. |
| | distilled water for injection | q.s. |
| | | 1.00 mL |

### Example 7

### Formulation Example 7 (tablet)

A tablet having the following composition is prepared by a conventional method. Compound a-93 (40 g), lactose (286.8 g) and potato starch (60 g) are mixed, and a 10% aqueous hydroxypropylcellulose solution (120 g) is added thereto. The mixture is kneaded, granulated, dried, and sieved to give granules for tableting by a conventional method. Magnesium stearate (1.2 g) is added and mixed, and the mixture is tableted by a tableting machine (RT-15 manufactured by Kikusui Seisakusho Ltd.) with a 8 mm diameter punch to give a tablet (containing 20 mg of active ingredient per tablet).

**[Table 22]**

| Formulation | | |
|---|---|---|
| | compound a-93 | 20 mg |
| | lactose | 143.4 mg |
| | potato starch | 30 mg |
| | hydroxypropylcellulose | 6 mg |
| | magnesium stearate | 0.6 mg |
| | | 200 mg |

### Example 8

### Formulation Example 8 (injection)

An injection having the following composition is prepared by a conventional method. Compound a-93 (1 g) and D-mannitol (5 g) are added to distilled water for injection and mixed. Hydrochloric acid and aqueous sodium hydroxide solution are further added to adjust the mixture to pH 6, and distilled water for injection is added to the total amount of 1000 mL. The obtained mixture is aseptically filled in a glass vial by 2 mL to give an injection (containing 2 mg of active ingredient per vial).

**[Table 23]**

| Formulation | | |
|---|---|---|
| | compound a-93 | 2 mg |
| | D-mannitol | 10 mg |
| | hydrochloric acid | q.s. |
| | aqueous sodium hydroxide solution | q.s. |
| | distilled water for injection | q.s. |
| | | 2.00 mL |

### Example 9

### Formulation Example 9 (eye drop)

An eye drop having the following composition is prepared by a conventional method. Compound a-93 (1 g) and sodium chloride (9 g) are added to distilled water for injection and mixed. Hydrochloric acid and aqueous sodium hydroxide solution are further added to adjust the mixture to pH 7, and distilled water for injection is added to the total amount of 1000 mL. The obtained mixture is aseptically filled in an instillation container by 1 mL to give an eye drop (containing 1 mg of active ingredient per container).

**[Table 24]**

| | | |
|---|---|---|
| Formulation | | |
| | compound a-93 | 1 mg |
| | sodium chloride | 9 mg |
| | hydrochloric acid | q.s. |
| | aqueous sodium hydroxide solution | q.s. |
| | distilled water for injection | q.s. |
| | | 1.00 mL |

### Industrial Applicability

According to the present invention, a therapeutic agent for ocular inflammation and the like comprising a pyrazolopyrimidine compound or a pharmaceutically acceptable salt thereof as an active ingredient is provided.

The prophylactic and/or therapeutic agent for ocular inflammatory diseases to be provided by the present invention can be used for the treatment and/or prophylaxis of ocular inflammatory diseases (for example, eyelid dermatitis, keratoconjunctivitis, scleritis, conjunctivitis {for example, allergic conjunctival diseases [for example, atopic keratoconjunctivitis, spring catarrh, giant papillary conjunctivitis, allergic conjunctivitis (for example, seasonal allergic conjunctivitis such as pollinosis and the like, perennial allergic conjunctivitis and the like) and the like] and the like} and the like).

## Claims

1. A prophylactic and/or therapeutic agent for ocular inflammatory diseases, comprising a pyrazolopyrimidine compound represented by the formula (I) 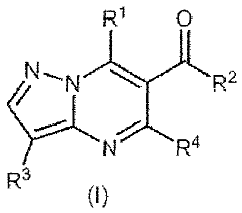 (wherein R¹ represents -NR^{1a}R^{1b} (wherein R^{1a} and R^{1b} are the same or different and each represents a hydrogen atom, lower alkyl optionally having substituent(s), cycloalkyl optionally having substituent(s), lower alkoxy optionally having substituent(s), lower alkanoyl optionally having substituent(s), aryl optionally having substituent(s), aralkyl optionally having substituent(s), an aromatic heterocyclic group optionally having substituent(s) or an aliphatic heterocyclic group optionally having substituent(s), or R^{1a} and R^{1b} form, together with the adjacent nitrogen atom, a nitrogen-containing heterocyclic group optionally having substituent(s)), -OR^{1c} (wherein R^{1c} represents a hydrogen atom, lower alkyl optionally having substituent(s), cycloalkyl optionally having substituent(s), aryl optionally having substituent(s), aralkyl optionally having substituent(s), an aromatic heterocyclic group optionally having substituent(s) or an aliphatic heterocyclic group optionally having substituent(s)) or -SR^{1d} (wherein R^{1d} is as defined for the aforementioned R^{1c}),
R² represents 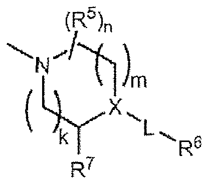 [wherein k and m each represents an integer of 0 - 2 (wherein the total of k and m is not more than 3),
n represents an integer of 0 - 4, and when n is 2, 3 or 4, respective R⁵ may be the same or different,
L represents a single bond, alkylene, C(=O) or SO₂,
R⁵ represents halogen, hydroxy, lower alkyl optionally having substituent(s) or lower alkoxy optionally having substituent(s),
R⁶ represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
X represents a nitrogen atom or -CR⁸ (wherein R⁸ represents a hydrogen atom, halogen, hydroxy, cyano, lower alkyl optionally having substituent(s) or lower alkoxy optionally having substituent(s), or forms a bond together with R⁷), and
R⁷ forms a bond together with R⁸, or represents a hydrogen atom, halogen, hydroxy, lower alkyl optionally having substituent(s) or lower alkoxy optionally having substituent(s)], 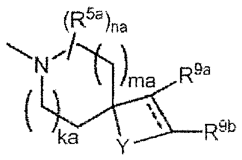 {wherein ka, ma and na are as defined for the aforementioned k, m and n, respectively,
R^{5a} represents as defined for the aforementioned R⁵,
--- is a single bond or a double bond,
R^{9a} and R^{9b} are the same or different and each represents a hydrogen atom or lower alkyl optionally having substituent(s), or R^{9a} and R^{9b} form, together with the respectively adjacent carbon atoms, an aliphatic ring optionally having substituent(s) or an aromatic ring optionally having substituent(s),
Y represents -CHR^{10a}-CHR^{10b}- (wherein R^{10a} and R^{10b} are the same or different and each represents a hydrogen atom, hydroxy, lower alkyl optionally having substituent(s) or lower alkoxy optionally having substituent(s), or R^{10a} and R^{10b} form, together with the respectively adjacent carbon atoms, an aliphatic ring optionally having substituent(s)), -CR^{10e}=CR^{10d}- (wherein R^{10c} and R^{10d} are the same or different and each represents a hydrogen atom or lower alkyl optionally having substituent(s), or R^{10c} and R^{10d} form, together with the respectively adjacent carbon atoms, an aliphatic ring having at least one double bond and optionally having substituent(s) or an aromatic ring optionally having substituent (s)), -Z^{a}-CR^{11a}R^{11b}- [wherein R^{11a} and R^{11b} are the same or different and each represents a hydrogen atom or lower alkyl optionally having substituent(s), or R^{11a} and R^{11b} form carbonyl together with the adjacent carbon atom, and Z^{a} represents C(=O), O, S, SO, SO₂ or NR¹² (wherein R¹² represents a hydrogen atom, lower alkyl optionally having substituent(s), lower alkanoyl optionally having substituent(s), lower alkoxycarbonyl optionally having substituent(s) or aralkyl optionally having substituent(s))], or -CR^{11c}R^{11d}-Z^{b}- (wherein R^{11c}, R^{11d} and Z^{b} are as defined for the aforementioned R^{11a}, R^{11b} and Z^{a}, respectively)}, or 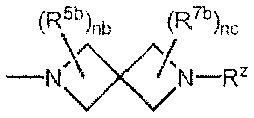 (wherein R^{z} represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
R^{5b} and R^{7b} are the same or different and each represents halogen, hydroxy, lower alkyl optionally having substituent(s) or lower alkoxy optionally having substituent(s),
nb represents an integer of 0 - 2, nc represents an integer of 0 - 2, when nb is 2, respective R^{5b}s are the same or different, when nc is 2, respective R^{7b}s are the same or different,
R³ represents -S(O)₂R^{13a} [wherein R^{13a} represents hydroxy, lower alkoxy optionally having substituent(s), -NR^{13b}R^{13c} (wherein R^{13b} and R^{13c} are the same or different and each represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), or R^{13b} and R^{13c} form, together with the adjacent nitrogen atom, a nitrogen-containing heterocyclic group optionally having substituent(s)), -NR^{13d}C(=O)R^{13e} (wherein R^{13d} represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), and R^{13e} represents a hydrogen atom, lower alkyl optionally having substituent(s), cycloalkyl optionally having substituent(s), aralkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s), N,N-di-arylamino optionally having substituent(s), aryl optionally having substituent(s), an aromatic heterocyclic group optionally having substituent(s) or an aliphatic heterocyclic group optionally having substituent(s)), -NR^{13f}C(=S)R^{13g} (wherein R^{13f} and R^{13g} are each as defined for R^{13d} and R^{13e} above, respectively), or -NR^{13h}S(O)₂R¹⁴ (wherein R^{13h} is as defined for R^{13d} above, and R¹⁴ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s))],
carboxy, lower alkoxycarbonyl optionally having substituent(s), lower alkyl optionally having substituent(s), lower alkanoyl optionally having substituent(s), aralkyl optionally having substituent(s), an aromatic heterocyclic group optionally having substituent(s), -C(=O)NR^{23a}R^{23b} [wherein R^{23a} and R^{23b} are the same or different and each represents a hydrogen atom, lower alkyl optionally having substituent(s), lower alkanoyl optionally having substituent(s), aralkyl optionally having substituent (s) or -S(O)₂R²⁴ (wherein R²⁴ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s)), or R^{23a} and R^{23b} form, together with the adjacent nitrogen atom, a nitrogen-containing heterocyclic group optionally having substituent(s)], or -NR^{23c}R^{23d} (wherein R^{23c} and R^{23d} are each as defined for R^{23a} and R^{23b} above, respectively),
R⁴ represents a hydrogen atom, halogen, lower alkyl optionally having substituent(s), aralkyl optionally having substituent(s), -NR^{15a}R^{15b} (wherein R^{15a} and R^{15b} are the same or different and each represents a hydrogen atom, lower alkyl optionally having substituent(s), cycloalkyl optionally having substituent(s), lower alkoxy optionally having substituent(s), lower alkanoyl optionally having substituent(s), aryl optionally having substituent(s), aralkyl optionally having substituent(s), an aromatic heterocyclic group optionally having substituent(s) or an aliphatic heterocyclic group optionally having substituent(s), or R^{15a} and R^{15b} form, together with the adjacent nitrogen atom, a nitrogen-containing heterocyclic group optionally having substituent(s)), -OR^{15c} (wherein R^{15c} represents a hydrogen atom, lower alkyl optionally having substituent(s), cycloalkyl optionally having substituent(s), aryl optionally having substituent(s), aralkyl optionally having substituent(s), an aromatic heterocyclic group optionally having substituent(s) or an aliphatic heterocyclic group optionally having substituent(s)), or -SR^{15d} (wherein R^{15d} is as defined for R^{15c} above)) or a pharmaceutically acceptable salt thereof, as an active ingredient.

2. The prophylactic and/or therapeutic agent according to claim 1, wherein R³ is -S (O)₂R^{13a} [wherein R^{13a} represents hydroxy, lower alkoxy optionally having substituent(s), -NR^{13b}R^{13c} (wherein R^{13b} and R^{13c} are the same or different and each represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), or R^{13b} and R^{13c} form, together with the adjacent nitrogen atom, a nitrogen-containing heterocyclic group optionally having substituent(s)), -NR^{13d}C (=O)R^{13e} (wherein R^{13d} represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s), and R^{13e} represents a hydrogen atom, lower alkyl optionally having substituent(s), cycloalkyl optionally having substituent(s), aralkyl optionally having substituent(s), lower alkoxy optionally having substituent(s), N-mono-lower alkylamino optionally having substituent(s), N,N-di-lower alkylamino optionally having substituent(s), N-cycloalkylamino optionally having substituent(s), N-mono-arylamino optionally having substituent(s), N,N-di-arylamino optionally having substituent(s), aryl optionally having substituent(s), an aromatic heterocyclic group optionally having substituent(s) or an aliphatic heterocyclic group optionally having substituent(s)), -NR^{13f}C (=S) R^{13g} (wherein R^{13f} and R^{13g} are as defined for R^{13d} and R^{13e} above, respectively), or -NR^{13h}S(O)₂R¹⁴ (wherein R^{13h} is as defined for R^{13d} above, and R¹⁴ represents lower alkyl optionally having substituent(s), cycloalkyl optionally having substituent(s), N-mono-lower alkylamino optionally having substituent(s), N,N-di-lower alkylamino optionally having substituent(s), aryl optionally having substituent(s), aralkyl optionally having substituent(s), an aromatic heterocyclic group optionally having substituent(s) or an aliphatic heterocyclic group optionally having substituent(s))].

3. A prophylactic and/or therapeutic agent for ocular inflammatory diseases, comprising a pyrazolopyrimidine compound represented by the formula (IA) 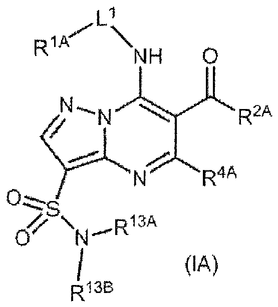 (wherein
L¹ represents a single bond or methylene,
R^{1A} represents lower alkyl optionally having substituent(s), aralkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
R^{2A} represents 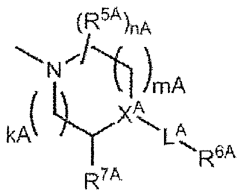 [wherein nA represents an integer of 0 - 2, and when nA is 2, respective R^{5A}s may be the same or different,
kA, mA and L^{A} are as defined for k, m and L above, respectively, R^{5A} represents halogen or lower alkyl,
R^{6A} represents cycloalkyl optionally having substituent(s),
aryl optionally having substituent(s), an aromatic heterocyclic group optionally having substituent(s) or an aliphatic heterocyclic group optionally having substituent(s),
X^{A} is a nitrogen atom or -CR^{8A} (wherein R^{8A} represents a hydrogen atom, halogen or lower alkyl, or forms a bond together with R^{7A}), and
R^{7A} forms a bond together with R^{8A}, or represents a hydrogen atom, halogen or lower alkyl], 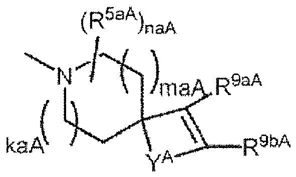 {wherein naA and R^{5aA} are as defined for nA and R^{5A} above, respectively,
maA and kaA are as defined for ma and ka above, respectively,
R^{9aA} and R^{9bA} form, together with the respectively adjacent carbon atoms, an aromatic ring optionally having substituent(s), Y^{A} represents -CHR^{10aA}-CHR^{10bA}- (wherein R^{10aA} and R^{10bA} are the same or different and each represents a hydrogen atom, hydroxy, lower alkyl optionally having substituent(s) or lower alkoxy optionally having substituent (s)), -CR^{10cA}=CR^{10dA}- (wherein R^{10cA} and R^{10dA} are the same or different and each represents a hydrogen atom or lower alkyl optionally having substituent(s)), -Z^{aA}-CR^{11aA}R^{11bA}- [wherein R11^{aA} and R^{11bA} are the same or different and each represents a hydrogen atom or lower alkyl optionally having substituent(s), or R^{11aA} and R^{11bA} form carbonyl together with the adjacent carbon atom, and Z^{aA} represents C(=O), O, S, SO, SO₂ or NR^{12A} (wherein R^{12A} represents a hydrogen atom, lower alkyl optionally having substituent(s), lower alkanoyl optionally having substituent(s), lower alkoxycarbonyl optionally having substituent(s) or aralkyl optionally having substituent(s))], or -CR^{11cA}R^{11dA}-Z^{bA}- (wherein R^{11cA}, R^{11dA} and Z^{bA} are as defined for R^{11aA}, R^{11bA} and Z^{aA} above, respectively)}, or 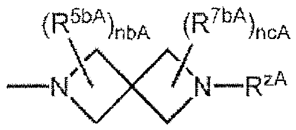 (wherein R^{zA} represents lower alkyl optionally having substituent(s), cycloalkyl optionally having substituent(s), aryl optionally having substituent(s), an aromatic heterocyclic group optionally having substituent(s) or an aliphatic heterocyclic group optionally having substituent(s),
R^{5bA} and R^{7bA} are the same or different and each represents halogen, hydroxy, lower alkyl optionally having substituent(s) or lower alkoxy optionally having substituent(s),
nbA represents an integer of 0 - 2, ncA represents an integer of 0 - 2, when nbA is 2, respective R^{5bA}s are the same or different and when ncA is 2, respective R^{7bA}s are the same or different),
R^{13A} represents a hydrogen atom, lower alkyl optionally having substituent(s), cycloalkyl optionally having substituent(s), aralkyl optionally having substituent(s), aryl optionally having substituent(s), an aromatic heterocyclic group optionally having substituent(s) or an aliphatic heterocyclic group optionally having substituent(s),
R^{13B} represents a hydrogen atom, lower alkyl optionally having substituent(s), lower alkoxy optionally having substituent(s), cycloalkyl optionally having substituent(s), aralkyl optionally having substituent(s), aryl optionally having substituent(s), an aromatic heterocyclic group optionally having substituent(s), an aliphatic heterocyclic group optionally having substituent (s) or COR^{13e1} (wherein R^{13e1} is as defined for R^{13e} above), or R^{13B} and R^{13A} form, together with the adjacent nitrogen atom, a nitrogen-containing heterocyclic group optionally having substituent(s),
R^{4A} represents a hydrogen atom or lower alkyl optionally having substituent(s)) or a pharmaceutically acceptable salt thereof, as an active ingredient.

4. The prophylactic and/or therapeutic agent according to claim 3, wherein L¹ is a single bond.

5. The prophylactic and/or therapeutic agent according to claim 3 or 4, wherein R^{1A} is aryl optionally having substituent(s).

6. The prophylactic and/or therapeutic agent according to claim 3 or 4, wherein R^{1A} is phenyl optionally having substituent(s).

7. The prophylactic and/or therapeutic agent according to any of claims 3 to 6, wherein R^{4A} is a hydrogen atom.

8. The prophylactic and/or therapeutic agent according to any of claims 3 to 7, wherein R^{13A} is a hydrogen atom, and R^{13B} is lower alkyl optionally having substituent(s).

9. The prophylactic and/or therapeutic agent according to any of claims 3 to 7, wherein R^{13A} is a hydrogen atom, and R^{13B} is COR^{13e1} (wherein R^{13e1} is as defined above).

10. The prophylactic and/or therapeutic agent according to claim 9, wherein R^{13e1} is lower alkyl optionally having substituent(s), cycloalkyl optionally having substituent(s), N-mono-lower alkylamino optionally having substituent(s) or N-mono-arylamino optionally having substituent(s).

11. The prophylactic and/or therapeutic agent according to any of claims 3 to 10, wherein R^{2A} is 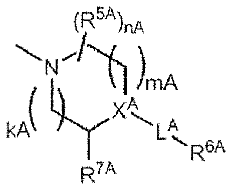 (wherein nA, mA, kA, R^{5A}, R^{6A}, R^{7A}, L^{A} and X^{A} are each as defined above, respectively).

12. The prophylactic and/or therapeutic agent according to claim 11, wherein R^{6A} is phenyl optionally having substituent(s).

13. The prophylactic and/or therapeutic agent according to claim 12, wherein nA is 0, kA and mA are each 1, R^{7A} is a hydrogen atom, L^{A} is a single bond, and X^{A} is CH.

14. The prophylactic and/or therapeutic agent according to any of claims 3 to 10, wherein R^{2A} is 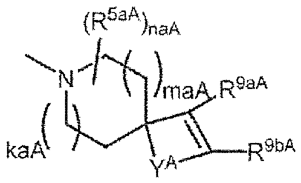 (wherein naA, maA, kaA, R^{5aA}, R^{9aA}, R^{9bA} and Y^{A} are each as defined above, respectively).

15. The prophylactic and/or therapeutic agent according to claim 14, wherein R^{9aA} and R^{9bA} form, together with the respectively adjacent carbon atoms, a benzene ring optionally having substituent(s), Y^{A} is -CHR^{10aA}-CHR^{10bA}- (wherein R^{10aA} and R^{10bA} are each as defined above, respectively), -CR^{10cA}=CR^{10dA}-(wherein R^{10cA} and R^{10dA} are each as defined above, respectively), -O-CR^{11aA}R^{11bA}- (wherein R^{11aA} and R^{11bA} are each as defined above, respectively), or -CR^{11cA}R^{11dA}-O- (wherein R^{11cA} and R^{11dA} are each as defined above, respectively).

16. The prophylactic and/or therapeutic agent according to any of claims 3 to 10, wherein R^{2A} is 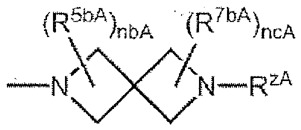 (wherein R^{zA}, R^{5bA}, R^{7bA}, nbA and ncA are each as defined above, respectively).

17. The prophylactic and/or therapeutic agent according to claim 16, wherein R^{zA} is phenyl optionally having substituent(s).

18. The prophylactic and/or therapeutic agent according to claim 1, wherein R¹ is -NHR^{1b}, said R^{1b} is phenyl optionally having substituent(s) selected from the group consisting of halogen and lower alkyl,
R² is 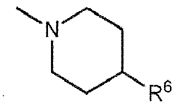 or 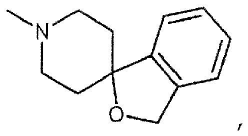 said R⁶ is phenyl optionally having halogen,
R³ is -S(O)₂R^{13a} or -C(=O)NH-S(O)₂-R²⁴, said R^{13a} is -NHR^{13c} or - NHC(=O)R^{13e}, said R^{13c} is lower alkoxy, said R^{13e} is lower alkyl or N-mono-lower alkylamino, and said R²⁴ is lower alkyl.

19. The prophylactic and/or therapeutic agent according to claim 18, wherein R^{1b} is phenyl optionally having substituent(s) selected from the group consisting of a fluorine atom, a chlorine atom and methyl.

20. The prophylactic and/or therapeutic agent according to claim 18, wherein R^{1b} is phenyl having two substituents selected from the group consisting of a fluorine atom, a chlorine atom and methyl.

21. The prophylactic and/or therapeutic agent according to any of claims 18 to 21, wherein R² is 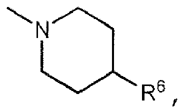 and said R⁶ is phenyl optionally having halogen.

22. The prophylactic and/or therapeutic agent according to claim 21, wherein R⁶ is phenyl optionally having a fluorine atom.

23. The prophylactic and/or therapeutic agent according to any of claims 18 to 22, wherein R³ is -S(O)₂R^{13a}, said R^{13a} is -NHR^{13c} or -NHC(=O)R^{13e}, said R^{13c} is lower alkoxy, and said R^{13e} is lower alkyl or N-mono-lower alkylamino.

24. The prophylactic and/or therapeutic agent according to claim 23, wherein R^{13c} is C₁₋₄ alkoxy, and R^{13e} is C₁₋₄ alkyl or N-mono-C₁₋₄ alkylamino.

25. The prophylactic and/or therapeutic agent according to any of claims 18 to 22, wherein R³ is -C(=O)NH-S(O)₂-R²⁴, and said R²⁴ is lower alkyl.

26. The prophylactic and/or therapeutic agent according to claim 25, wherein R²⁴ is C₁₋₄ alkyl.

27. The prophylactic and/or therapeutic agent according to claim 18, wherein the pyrazolopyrimidine compound is a compound selected from the group consisting of
7-(2-chloro-5-methylphenylamino)-N-(ethylcarbamoyl)-6-[4-(4-fluorophenyl)piperidine-1-carbonyl]pyrazolo[1,5-a]pyrimidine-3-sulfonamide,
N-{7-(2,5-dichlorophenylamino)-6-[4-(4-fluorophenyl)piperidine-1-carbonyl]pyrazolo[1,5-a]pyrimidin-3-ylsulfonyl}propionamide,
7-(2,5-dichlorophenylamino)-6-[4-(4-fluorophenyl)piperidine-1-carbonyl]-N-methoxypyrazolo[1,5-a]pyrimidine-3-sulfonamide,
N-{7-(5-chloro-2-fluorophenylamino)-6-[4-(4-fluorophenyl)piperidine-1-carbonyl]pyrazolo[1,5-a]pyrimidin-3-ylsulfonyl}acetamide,
7-(5-chloro-2-fluorophenylamino)-N-(ethylcarbamoyl)-6-[4-(4-fluorophenyl)piperidine-1-carbonyl]pyrazolo[1,5-a]pyrimidine-3-sulfonamide,
N-{7-(2,5-difluorophenylamino)-6-[4-(4-fluorophenyl)piperidine-1-carbonyl]pyrazolo[1,5-a]pyrimidin-3-ylsulfonyl}acetamide,
7-(2-chloro-5-methylphenylamino)-N-(ethylcarbamoyl)-6-(3H-spiro[isobenzofuran-1,4'-piperidin]-1'-yl carbonyl)pyrazolo[1,5-a]pyrimidine-3-sulfonamide,
N-[7-(4-fluoro-2-methylphenylamino)-6-(4-phenylpiperidine-1-carbonyl)pyrazolo[1,5-a]pyrimidine-3-carbonyl]ethanesulfonamide,
N-{7-(2-fluoro-5-methylphenylamino)-6-[4-(4-fluorophenyl)piperidine-1-carbonyl]pyrazolo[1,5-a]pyrimidine-3-carbonyl}ethanesulfonamide, and
N-{7-(4-fluoro-2-methylphenylamino)-6-[4-(4-fluorophenyl)piperidine-1-carbonyl]pyrazole[1,5-a]pyrimidine-3-carbonyl}ethanesulfonamide.

28. The prophylactic and/or therapeutic agent according to any of claims 1 to 27, wherein the ocular inflammatory disease is a disease selected from conjunctivitis, eyelid dermatitis, keratoconjunctivitis and scleritis.

29. The prophylactic and/or therapeutic agent according to any of claims 1 to 27, wherein the ocular inflammatory disease is conjunctivitis.

30. The prophylactic and/or therapeutic agent according to claim 29, wherein the conjunctivitis is an allergic conjunctival disease.

31. The prophylactic and/or therapeutic agent according to claim 30, wherein the allergic conjunctival disease is a disease selected from allergic conjunctivitis, atopic keratoconjunctivitis, spring catarrh and giant papillary conjunctivitis.

32. The prophylactic and/or therapeutic agent according to claim 31, wherein the allergic conjunctivitis is a disease selected from seasonal allergic conjunctivitis and perennial allergic conjunctivitis.

33. The prophylactic and/or therapeutic agent according to any of claims 1 to 27, which is used for itching, redness, edema and ulcer occurring as symptoms of ocular inflammatory diseases.

34. A method for the prophylaxis and/or treatment of an ocular inflammatory disease, comprising a step of administering an effective amount of the pyrazolopyrimidine compound according to any of claims 1 to 27 or a pharmaceutically acceptable salt thereof.

35. The method according to claim 34, wherein the ocular inflammatory diseases is a disease selected from conjunctivitis, eyelid dermatitis, keratoconjunctivitis and scleritis.

36. The method according to claim 35, wherein the ocular inflammatory disease is conjunctivitis.

37. The method according to claim 36, wherein the conjunctivitis is an allergic conjunctival disease.

38. The method according to claim 37, wherein the allergic conjunctival disease is a disease selected from allergic conjunctivitis, atopic keratoconjunctivitis, spring catarrh and giant papillary conjunctivitis.

39. The method according to claim 38, wherein the allergic conjunctivitis is a disease selected from seasonal allergic conjunctivitis and perennial allergic conjunctivitis.

40. A method for the prophylaxis and/or treatment of itching, redness, edema and ulcer occurring as symptoms of ocular inflammatory diseases, comprising a step of administering an effective amount of the pyrazolopyrimidine compound according to any of claims 1 to 27 or a pharmaceutically acceptable salt thereof.

41. The pyrazolopyrimidine compound according to any of claims 1 to 27 or a pharmaceutically acceptable salt thereof for use in the prophylaxis and/or treatment of ocular inflammatory diseases.

42. The pyrazolopyrimidine compound according to claim 41 or a pharmaceutically acceptable salt thereof, wherein the ocular inflammatory diseases is a disease selected from conjunctivitis, eyelid dermatitis, keratoconjunctivitis and scleritis.

43. The pyrazolopyrimidine compound according to claim 41 or a pharmaceutically acceptable salt thereof, wherein the ocular inflammatory diseases is conjunctivitis.

44. The pyrazolopyrimidine compound according to claim 43 or a pharmaceutically acceptable salt thereof, wherein the conjunctivitis is an allergic conjunctival disease.

45. The pyrazolopyrimidine compound according to claim 44 or a pharmaceutically acceptable salt thereof, wherein the allergic conjunctival disease is a disease selected from allergic conjunctivitis, atopic keratoconjunctivitis, spring catarrh and giant papillary conjunctivitis.

46. The pyrazolopyrimidine compound according to claim 45 or a pharmaceutically acceptable salt thereof, wherein the allergic conjunctivitis is a disease selected from seasonal allergic conjunctivitis and perennial allergic conjunctivitis.

47. The pyrazolopyrimidine compound according to any of claims 1 to 27 or a pharmaceutically acceptable salt thereof for use in the prophylaxis and/or treatment of itching, redness, edema and ulcer occurring as symptoms of ocular inflammatory diseases.

48. Use of the pyrazolopyrimidine compound according to any of claims 1 to 27 or a pharmaceutically acceptable salt thereof for the manufacture of a prophylactic and/or therapeutic agent for ocular inflammatory diseases.

49. The use according to claim 48, wherein the ocular inflammatory disease is a disease selected from conjunctivitis, eyelid dermatitis, keratoconjunctivitis and scleritis.

50. The use according to claim 48, wherein the ocular inflammatory disease is conjunctivitis.

51. The use according to claim 50, wherein the conjunctivitis is an allergic conjunctival disease.

52. The use according to claim 51, wherein the allergic conjunctival disease is a disease selected from allergic conjunctivitis, atopic keratoconjunctivitis, spring catarrh and giant papillary conjunctivitis.

53. The use according to claim 52, wherein the allergic conjunctivitis is a disease selected from seasonal allergic conjunctivitis and perennial allergic conjunctivitis.

54. Use of the pyrazolopyrimidine compound describen in any of claims 1 to 27 or a pharmaceutically acceptable salt thereof for the manufacture of a prophylactic and/or therapeutic agent for itching, redness, edema and ulcer occurring as symptoms of ocular inflammatory diseases.
